# EUROPEAN PATENT SPECIFICATION

(11) **EP 2 879 599 B1**
(45) Date of publication and mention of the grant of the patent: **29.04.2020**
(21) Application number: 13825135.0
(22) Date of filing: 31.07.2013
(51) Int. Cl.: A61M 25/06, A61M 25/04, A61M 25/00

(54) **ACCESS CLOSURE CONFIGURATION**
ZUGANGSVERSCHLUSSKONFIGURATION
CONFIGURATION DE FERMETURE D'ACCÈS

(30) Priority: 01.08.2012 US 201261678306 P
(43) Date of publication of application: 10.06.2015
(73) Proprietor: Arstasis, Inc., Redwood City, CA 94063 (US)
(72) Inventor: MODESITT, D., Bruce, San Carlos, CA 94070 (US); PARASCHAC, Joseph, F., Campbell, 95008 (US); ELLINGWOOD, Brian, A., Sunnyvale, CA 94085 (US)
(74) Representative: Lecomte & Partners
(86) International application number: PCT/US2013/052926
(87) International publication number: WO 2014/022509

(56) References cited:
- US-A- 5 810 849
- US-A1- 2005 267 520
- US-A1- 2006 271 078
- US-A1- 2009 005 793
- US-A1- 2011 125 178
- US-A1- 2011 208 215
- US-A1- 2011 230 906

## Description

### FIELD OF THE INVENTION

The present invention relates to the field of accessing a biological lumen and closing the access pathway or tract thereby created.

### BACKGROUND

A number of diagnostic and interventional vascular procedures are now performed translumenally, where a catheter is introduced to the vascular system at a convenient access location, such as the femoral, brachial, radial, or subclavian arteries, and guided through the vascular system to a target location to perform therapy or diagnosis. When vascular access is no longer required, the catheter and other vascular access devices must be removed from the vascular entrance and bleeding at the puncture site must be stopped. One common approach for providing hemostasis at this site is to apply external force near and upstream from the puncture site, typically by what is known as "manual compression" technique. This hemostasis technique is time-consuming, frequently requiring one-half hour or more of compression before hemostasis. This procedure is uncomfortable for the patient and frequently requires administering analgesics. Excessive pressure can also present the risk of total occlusion of the blood vessel, resulting in ischemia and/or thrombosis. After hemostasis is achieved by manual compression, the patient typically is required to remain recumbent for six to eighteen hours under observation to assure continued hemostasis. During this time bleeding from the vascular access wound can restart potentially resulting in major complications. These complications may require blood transfusion and/or surgical intervention.

Bioabsorbable fasteners have also been used to stop bleeding. Generally, these approaches rely on the placement of a thrombogenic and bioabsorbable material, such as collagen, at the superficial arterial wall over the puncture site. This method generally presents difficulty locating the interface of the overlying-tissue and the adventitial surface of the blood vessel. Implanting the fastener too far from the desired location can result in failure to provide hemostasis. If, however, the fastener intrudes into the vascular lumen, thrombus can form on the fastener. Thrombus can embolize downstream and/or block normal blood flow at the thrombus site. Implanted fasteners can also cause infection and auto-immune reactions/rejections of the implant.

Suturing methods also are used to provide hemostasis after vascular access. The suture-applying device typically is introduced through the tissue tract with a distal end of the device located at the vascular puncture. Needles in the device draw suture through the blood vessel wall on opposite sides of the punctures, and the suture is secured directly over the adventitial surface of the blood vessel wall to close the vascular access wound. To be successful, suturing methods typically need to be performed with a precise control; the associated needles need to be properly directed through the blood vessel wall so that the suture is well anchored in tissue to provide for tight closure. Suturing methods typically also require additional steps for the surgeon, interventionalist, or physician.

US patent application published US 2005/0267520 A1 discloses a device for forming an expandable tract across a wall of a blood vessel, according to the preamble of claim 1. US patent US 5,810,849 discloses a device for sealing an opening in an anatomical structure within a living body.

Due to the deficiencies of the above methods and devices, a need exists for a more reliable vascular closure configuration and technique. There also exists a need for a vascular closure device and method that is self-sealing and secure. There also exists a need for a vascular closure device and method requiring no or few extra steps to close the vascular site. Configurations are presented herein to address these challenges.

### SUMMARY

The invention is directed to a device for forming an expandable tract across a wall of a blood vessel, according to claim 1, comprising: a needle; an anchor assembly comprising a proximal portion having a handle, a flexible distal portion, and a pre-bent midportion intercoupled between the proximal and distal portions, the pre-bent midportion comprising an at least partially encapsulating saddle-shaped needle receiving structure configured to receive and support a needle that may be inserted through a portion of the proximal portion; wherein at least a distal tip of the flexible distal portion is configured to be placed within a lumen of the blood vessel through a first passage created across the wall with a sharpened member at a first angle relative to a lumen longitudinal axis defined by the lumen of the blood vessel in the region adjacent the first passage; and wherein upon applying a force to the anchor assembly to position an adjacent portion of the blood vessel wall into a desired contact configuration relative to the anchor assembly, the needle is operatively coupled to the anchor assembly such that it may be advanced across the wall of the blood vessel and into contact with the at least partially encapsulating saddle-shaped needle receiving structure, thereby creating an expandable tract between overlapping tissue portions of the vessel wall. The proximal portion may comprise an elongate tubular member through which the needle may be slidably coupled. The needle may comprise a hollow needle defining a working lumen therethrough. The needle may comprise a trocar or chisel tip geometry. The anchor assembly may be configured to direct the needle in a substantially straight trajectory across the wall of the blood vessel and into contact with the at least partially encapsulating saddle-shaped needle receiving structure. The anchor assembly and needle may be configured to direct the needle in an arcuate trajectory across the wall of the blood vessel and into contact with the at least partially encapsulating saddle-shaped needle receiving structure. The anchor assembly and needle may be configured to direct the needle in a two part trajectory across the wall of the blood vessel and into contact with the at least partially encapsulating saddle-shaped needle receiving structure, wherein a distal portion of the needle trajectory forms a distal portion of the expandable tract that is angled more steeply relative to the lumen longitudinal axis than is a proximal portion of the expandable tract. In another embodiment, a distal portion of the needle trajectory leading to the lumen of the blood vessel may be angled more shallowly relative to the lumen longitudinal axis than is the trajectory of the proximal portion of the needle. The anchor assembly and needle may be configured to direct the needle in a two part trajectory across the wall of the blood vessel and into contact with the at least partially encapsulating saddle-shaped needle receiving structure such that proximal portion of the expandable tract is substantially parallel with the lumen longitudinal axis. The device further may comprise a load assisting member movably coupled to the anchor assembly configured to be controllably extended from the anchor assembly before applying the force to the anchor assembly. The load assisting member may be controllably rotatable about a pivot point relative to the anchor assembly. The load assisting member may be controllably insertable outward from an outer surface of the anchor assembly along a substantially straight axial pathway relative to the anchor assembly. The load assisting member may be controllably insertable outward from an outer surface of the anchor assembly along an arcuate pathway relative to the anchor assembly. The device further may comprise a proximal load applying member operatively coupled to the handle and configured to transfer a load from a proximal portion of the anchor assembly to one or more members coupled to the load assisting structure. The device further may comprise a guidewire insertable through the expandable tract. The guidewire may be inserted through at least a portion of the needle. The device further may comprise a dilating instrument insertable across the expandable tract. The flexible distal portion of the anchor assembly may comprise a wire formed into a longitudinal coil. The device further may comprise an elongate structural core wire positioned through a lumen defined through the longitudinal coil. The elongate structure core wire may comprise a noncircular cross sectional geometry configured to impart nonhomogeneous bending characteristics upon the flexible distal portion of the anchor assembly. The noncircular cross sectional geometry may comprise a rectangular cross sectional shape. A proximal end of the flexible distal portion of the anchor assembly may be removably coupled to a distal end of the prebent midportion. The proximal end of the flexible distal portion of the anchor assembly may be removably coupled to the distal end of the pre-bent midportion using a mechanical latch fitting. The anchor assembly and needle may be configured to create the expandable tract to have a geometry relative to the wall of the blood vessel such that upon withdrawal of structures from the expandable tract, blood pressure acting on the vessel wall causes the overlapping tissue portions to collapse against each other and self-seal the expandable tract. The at least partially encapsulating saddle-shaped needle receiving structure may comprise a laser-cut tubular member portion. The at least partially encapsulating saddle-shaped needle receiving structure may comprise a mechanically-formed tubular member portion. The at least partially encapsulating saddle-shaped needle receiving structure may have a geometry configured to encapsulate about ½ of the surface geometry of a distal tip of the needle when interfaced with the needle.

### BRIEF DESCRIPTION OF THE DRAWINGS

Figures 1A-1U illustrate schematic representations of one embodiment of a lumen access and closure configuration in accordance with the present invention, at various stages of deployment.
Figures 2A-2T illustrate three-dimensional assemblies and subassemblies in accordance with various embodiments of lumen access and closure configurations of the present invention, in various stages of deconstruction.
Figure 3 illustrates one embodiment of a lumen access and closure technique in accordance with the present invention.
Figure 4 illustrates one embodiment of a lumen access and closure technique in accordance with the present invention.
Figures 5A and 5B illustrate embodiments of single-segmented access tracts created in accordance with the present invention.
Figures 6A and 6B illustrate embodiments of single-segmented access tracts created in accordance with the present invention.
Figures 7A and 7B illustrate embodiments of single-segmented access tracts created in accordance with the present invention.
Figures 8A-8C illustrate three-dimensional assemblies and subassemblies in accordance with various embodiments of lumen access and closure configurations of the present invention, in various stages of deconstruction.

### DETAILED DESCRIPTION

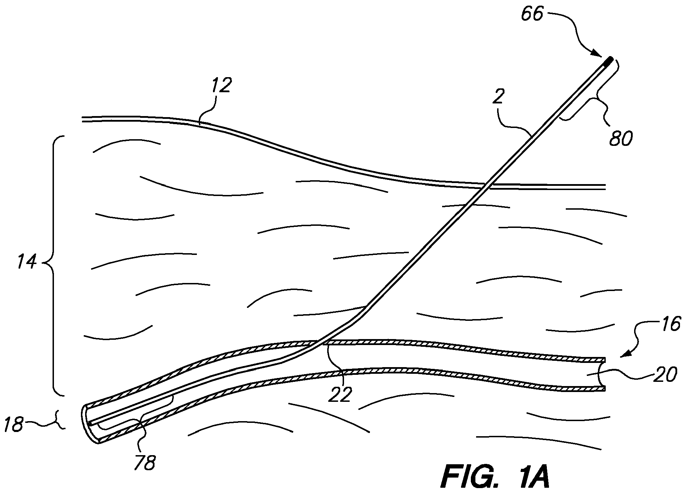

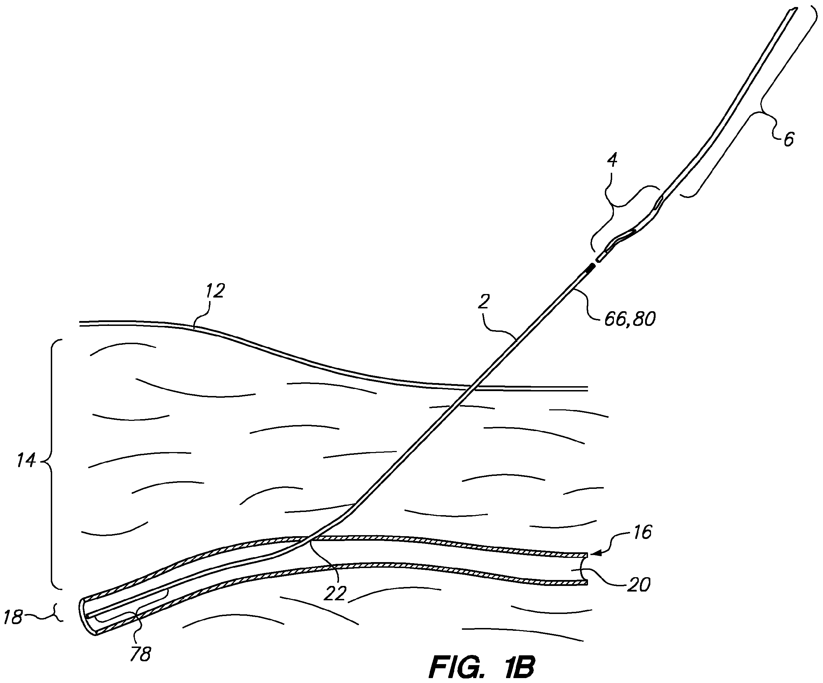

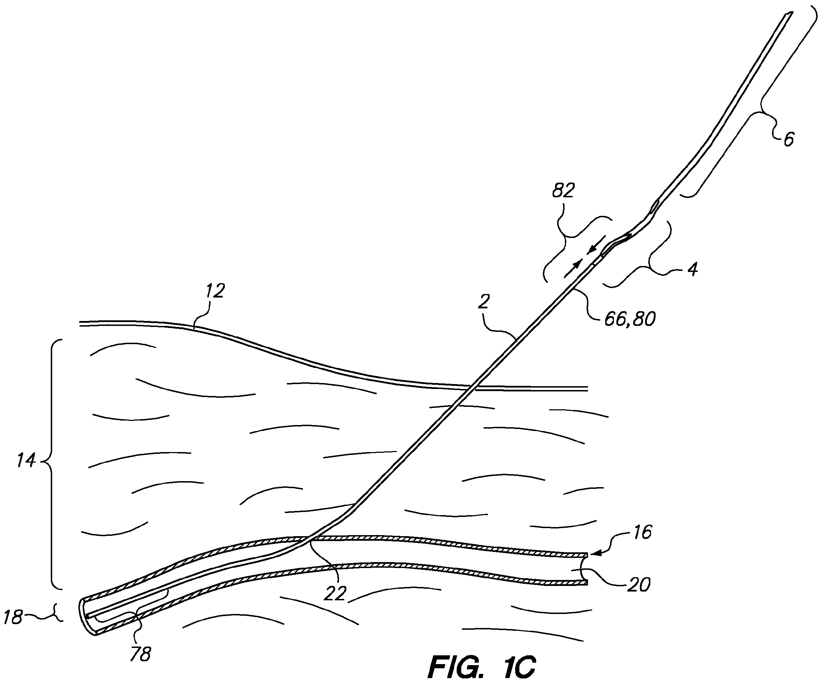

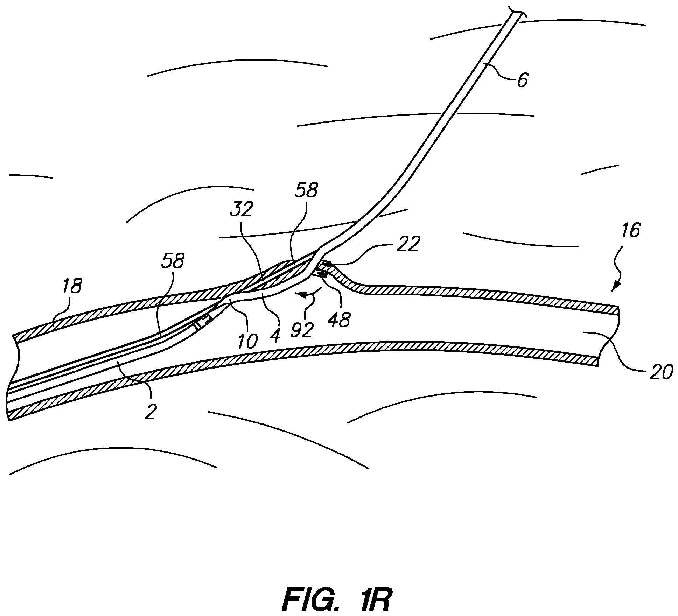

Referring to Figure 1A, a group of associated tissue structures is illustrated, with a skin layer (12), subdermal tissue layer (14), and blood vessel (16) depicted. The blood vessel (16) is shown in cross sectional form with the vessel walls (18) sectioned and the vascular lumen (20) shown. As shown in Figure 1A, a flexible distal portion (2) of an anchor assembly has been placed such that a distal portion (78) resides within the vessel lumen (20) where blood would be flowing, and a proximal end (80) resides external to the patient. In one embodiment, such a flexible elongate member (2) maybe placed using a Seldinger technique, wherein a sharpened object, such as a scalpel, may be utilized to create a small entry point through the skin layer (12), after which a hollow needle may be inserted through the entry point, across the subdermal layers (14), and into the targeted vessel lumen (20) through a first passage (22), the passage generally created by the needle in a Seldinger technique configuration, followed by insertion of the elongate flexible member (2), which may resemble a guidewire in structure and/or function. The proximal aspect (80) of the depicted flexible member (2) comprises a coupling fitting (66) configured to be coupled, or removably coupled, to another portion of an anchor assembly structure, as shown, for example, in Figure 1B. Referring to Figure 1B, the coupling fitting (66) of the elongate flexible member (2) is being compressed against a distal portion of the depicted pre-bent midportion (4) of the depicted anchor assembly (the assembly ultimately comprising the elongate flexible member 2, the midportion 4, and the proximal portion 6, which may also feature a proximal manipulation handle 8; a needle member 40 may also be coupled to the assembly, as described below) to result in a coupled assembly, as shown in Figure 1C.

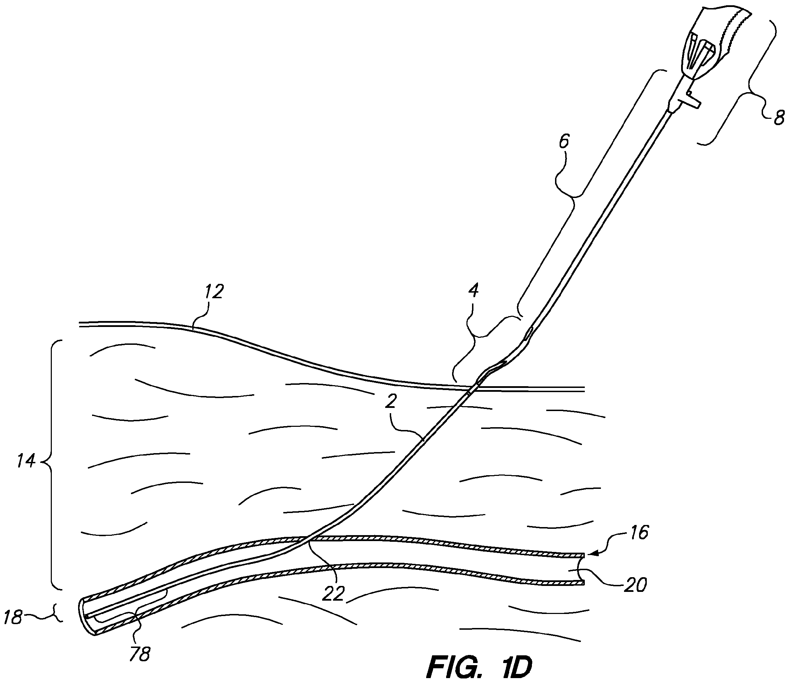

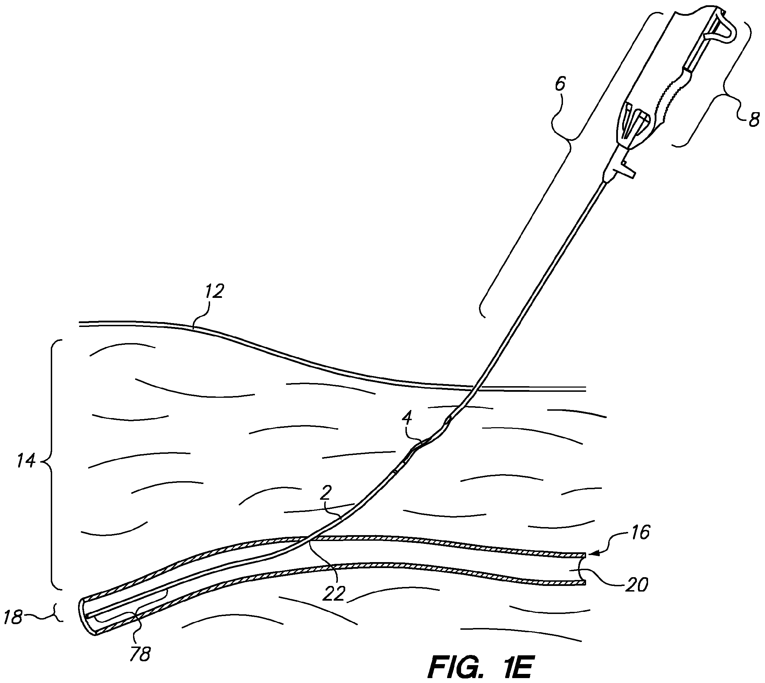

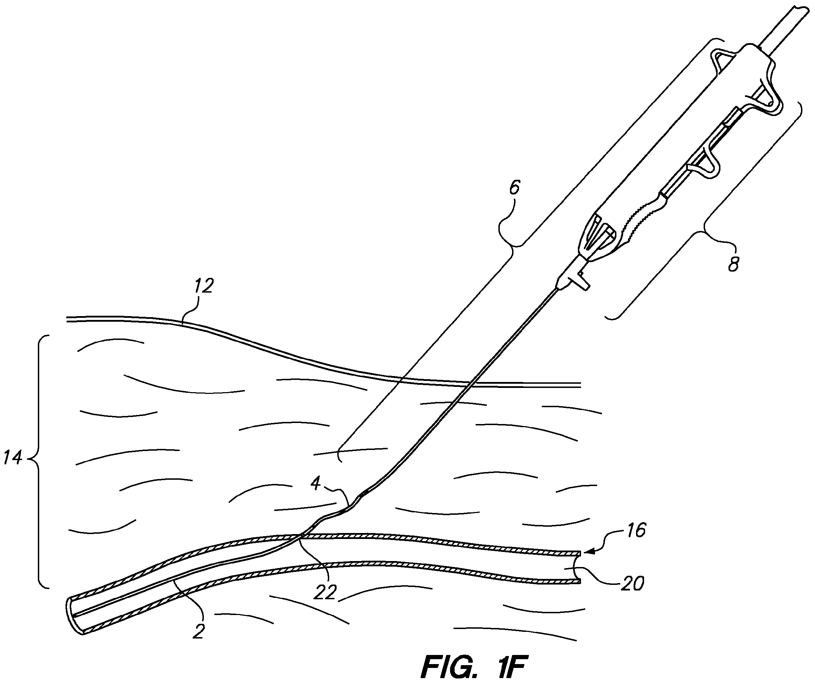

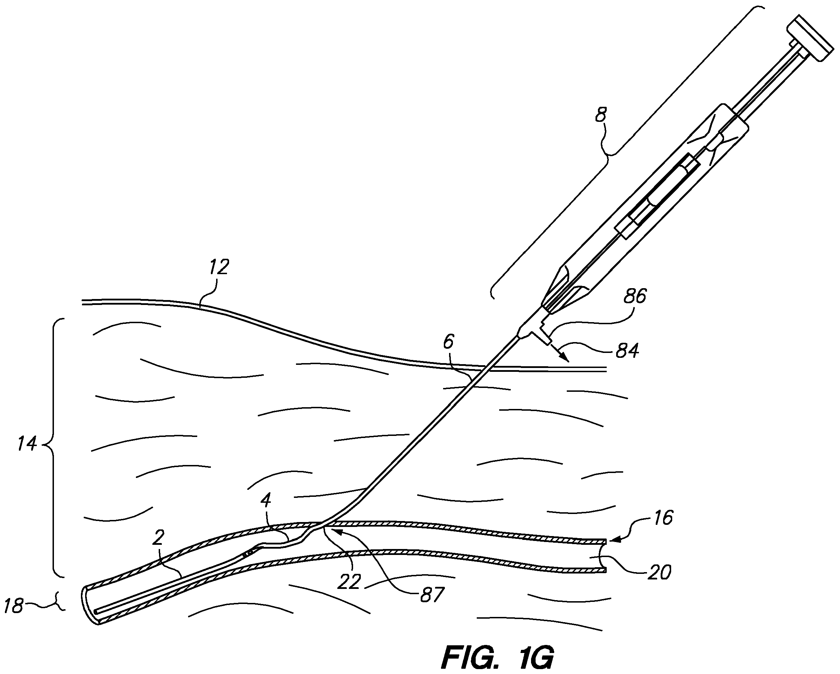

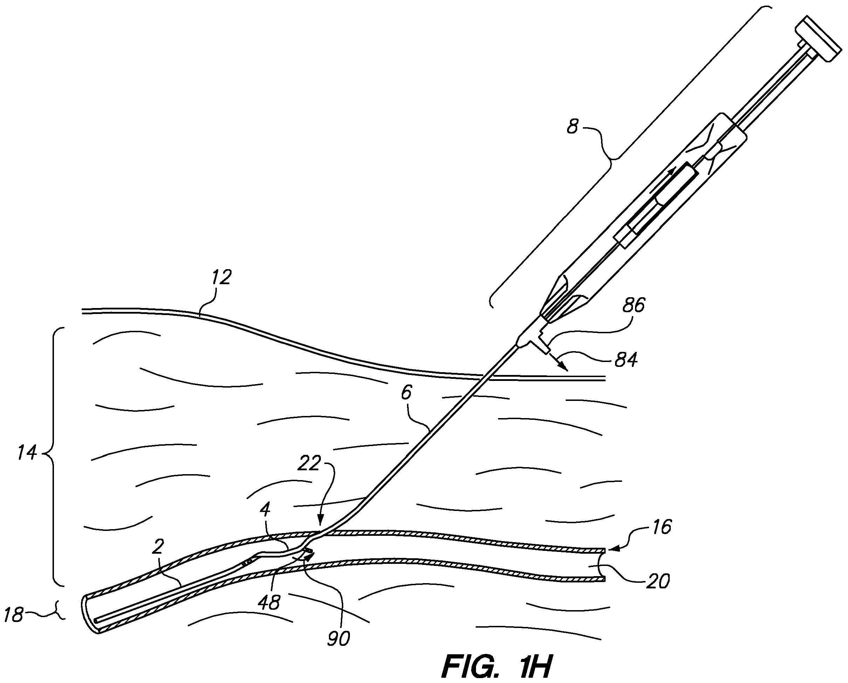

Referring to Figures 1D, 1E, and 1F, with the assembly (2, 4, 6, 8) in a coupled formation, it may be inserted further into the patient in an elongate fashion as shown, with the distal end (78) of the flexible distal portion (2) continuing to insert farther into the vessel lumen (20). Referring to Figure 1G, when the proximal aspect of the prebent midportion (4) enters the blood contained within the vascular lumen (20), a small distal port (say in the region of depicted element 87) that is fluidly coupled (i.e., by a "marker channel" or lumen defined through the interconnecting portion of the proximal portion 6 of the assembly) with a proximal blood marker port (86) mounted to the distal end of the handle (8) allows a small flow of blood to travel up the marker channel from the distal port (87) and out (84 - the expressed mark of blood itself) of the proximal marker port (86) where it intentionally is viewable by the operator holding the handle (8). In one embodiment, the lumen through which the needle travels in the anchor assembly proximal portion (6) functions as the blood marker channel (i.e., such lumen functions to contain the needle and also to fluidly connect the proximal and distal marker ports). In another embodiment, a specific lumen may be configured to function as a marker channel. Such a blood marking is an indicator that the distal port location (87) of the assembly has reached the vessel lumen (20). Referring to Figure 1H, with the visible mark of blood (84) proximally through the proximal port (86), the operator may mechanically deploy a load assisting member (48), causing such member to extend away from the assembly midportion (4) and provide a greater net load bearing surface configuration to assist with pullback loading, as described above. Such mechanical deployment may be controlled by intentional controlled movement of a small handle or trigger movably coupled to the proximal handle (8), which may cause a coupled tensile element such as a wire to apply a tensile load upon an aspect of the load assisting member (48), causing it to rotate out (90), as shown in Figure 1H, insert out somewhat linearly along an axis, or insert/rotate in a combined or arcuate fashion. In one embodiment, the blood marking described above (a visible mark of blood 84 out of proximal marking port 86) may be utilized to ensure that the load assisting member (48) will be close to the vessel wall (18) when deployed, to prevent undesired relative motion of the load assisting member (48) with other tissue surfaces, such as the opposing vessel wall.

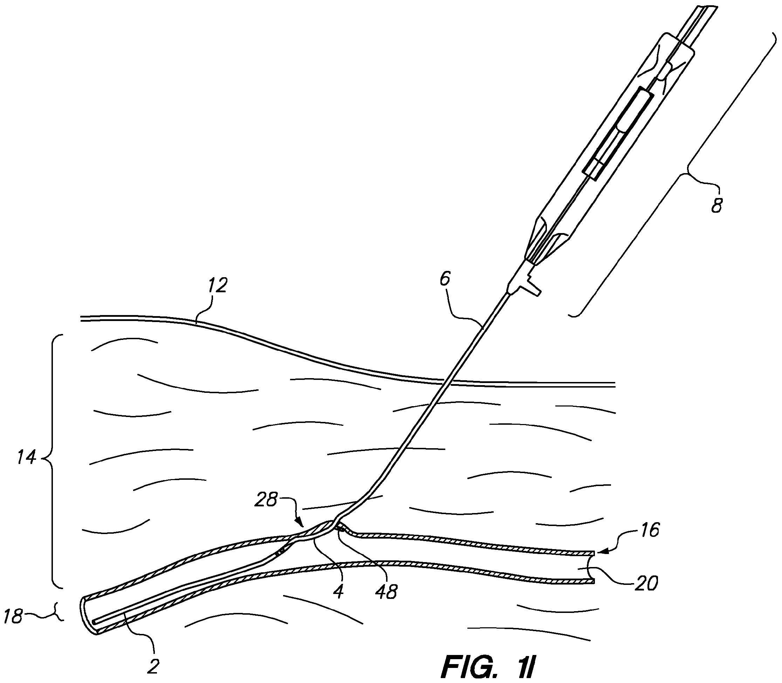

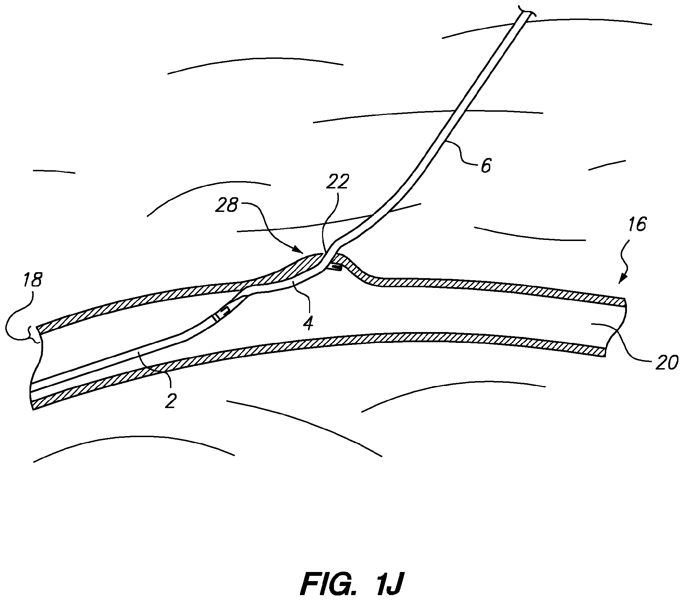

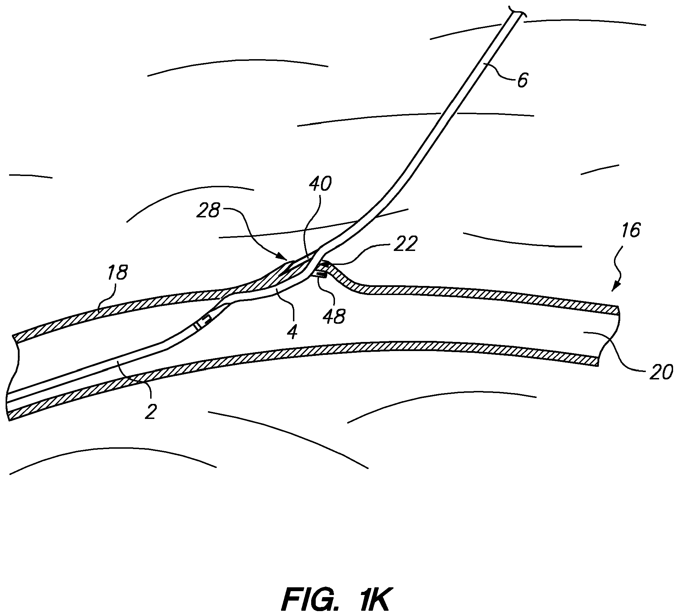

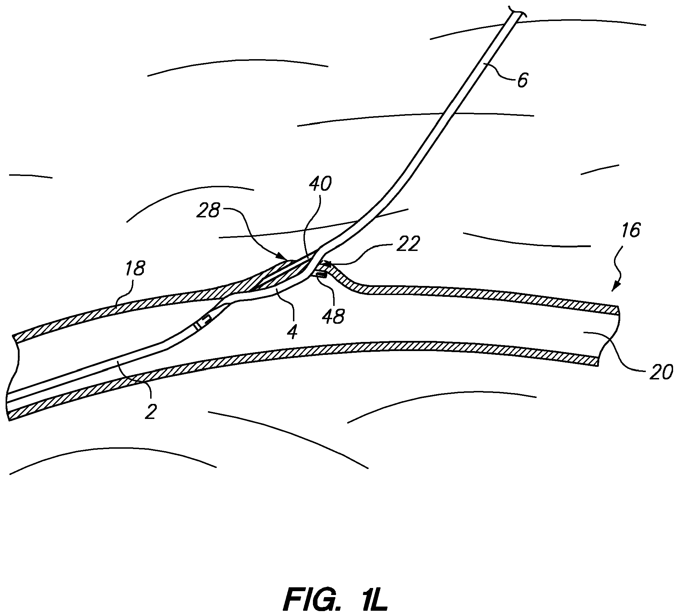

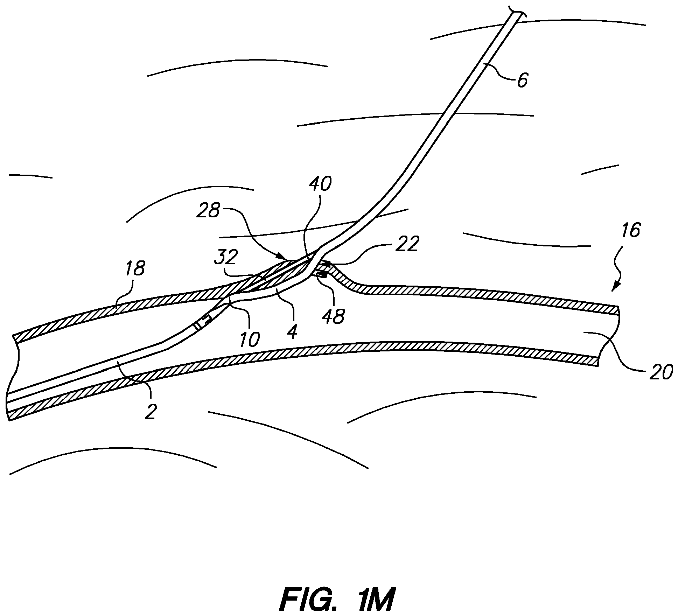

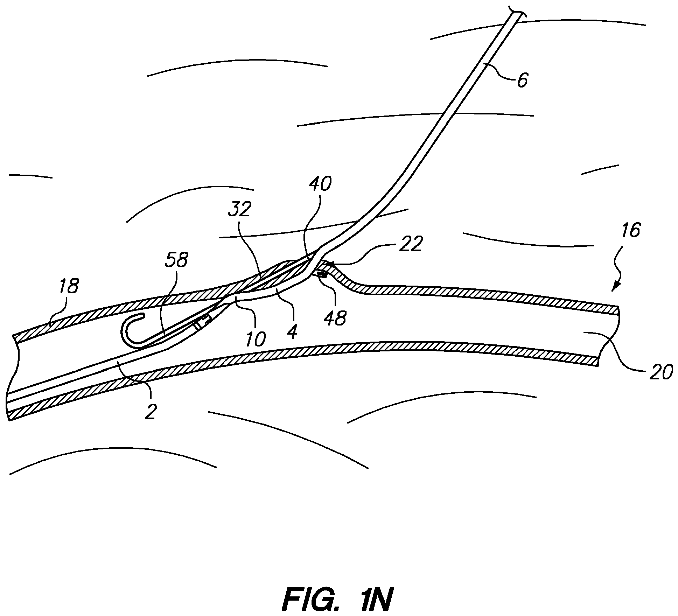

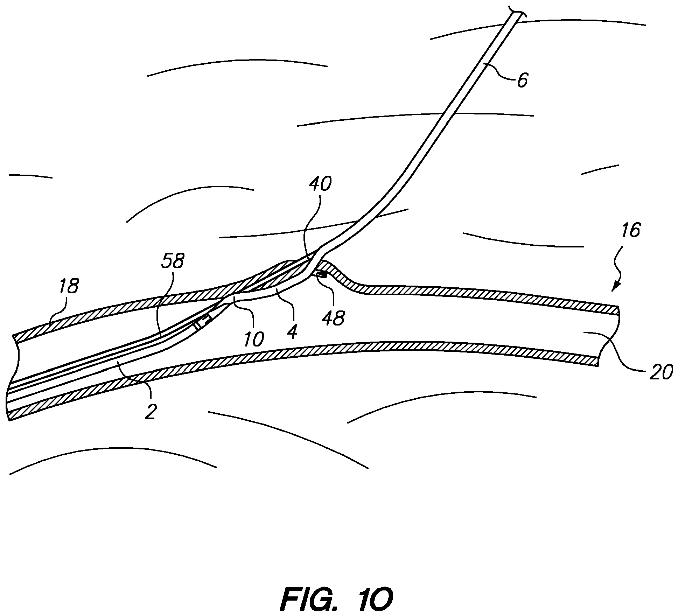

Referring to Figure 1I, with the load assisting member (48) extended out, a proximal load may be delivered to the assembly through the handle (8) and/or proximal shaft (6) to cause the prebent midportion (4) and load assisting member (48) to urge the nearby portion of the vascular wall (18) into a specific desired contact configuration (28). In one embodiment, in a desired contact configuration (28), the nearby portion of the vascular wall is folded around and urged against the prebent midportion, somewhat akin to the manner in which a towel hangs over a horizontal towel rod in a bathroom due to gravity-based loading. Figure 1J shows a closer view of the desired contact configuration (28) featured in Figure 1I. Referring to Figure 1K, the importance of the shape of the prebent midportion and the desired contact configuration (28) are emphasized with insertion of a needle member (40) across the adjacent vessel wall portion (18) to create an expandable tract. In the depicted embodiment, the needle member (40) is movably coupled through a port and lumen defined through the proximal member (6) which leads to a plunger (96) that is movably coupled to the handle (8), enabling an operator to move the needle in insertion and retraction while also retaining the desired contact configuration (28). Figure 1L shows further insertion of the needle member (40). Figure 1M shows full insertion of the needle member (40) into a configuration wherein the distal portion of the needle is placed into contact with a saddle-shaped surface of the prebent midportion (4) which serves to constrain motion of the needle (40). Indeed, in one embodiment, the prebent midportion (4) is specifically designed to have a canoe-like geometry configured to guide the needle (40) tip through a valley-like spine of the distal aspect of the prebent midportion (4) and into the configuration shown in Figure 1M wherein the needle (40) tip is safely and predictably motion-constrained. With the cutting action of the needle member (40) complete and the expandable tract (32) created through two now overlapping portions of the vessel wall (18; i.e., bisected by the expandable tract 32 and oriented at an angle relative to a longitudinal axis of the vessel wall or vessel lumen itself), another elongate flexible member, such as a guidewire (58), may be inserted through a lumen formed through the needle (40), as shown in Figure 1N. Referring to Figure 1O, more length of the guidewire (58) has been inserted with the needle (40) remaining in place movably coupled to the anchor assembly.

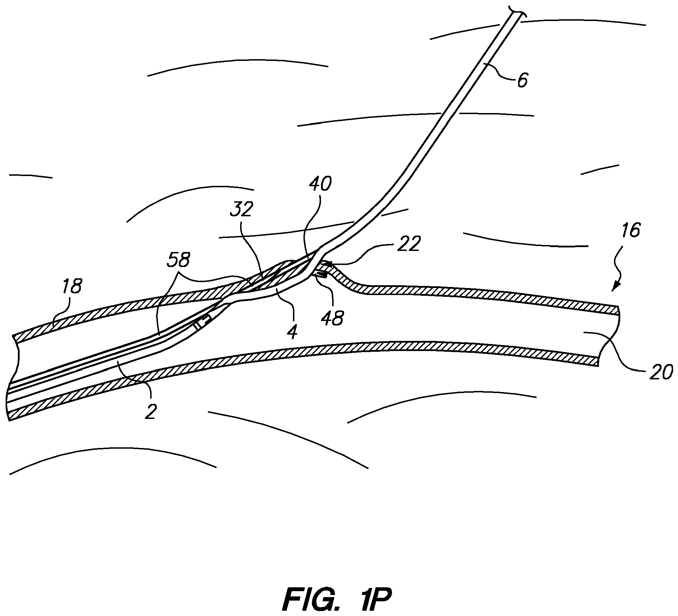

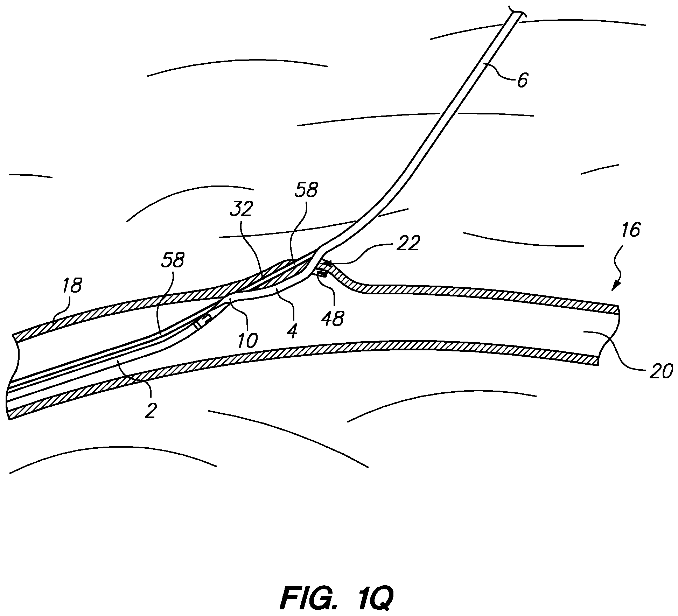

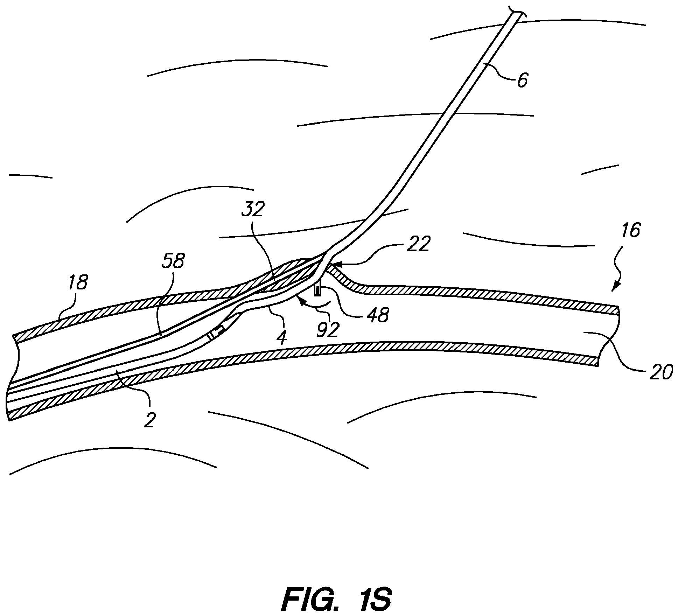

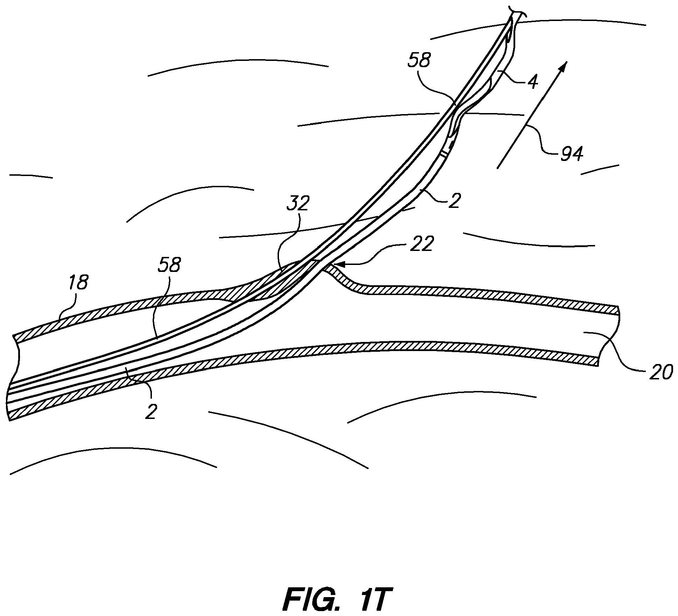

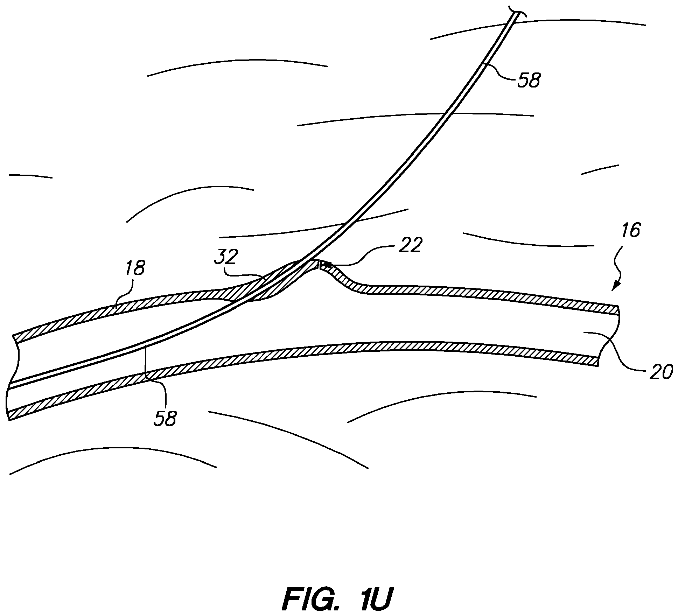

Referring to Figure 1P, with adequate desired length of the guidewire (58) inserted into the vascular lumen (20), the needle member (40) may be retracted (i.e., by using the movable needle insertion/retraction aspect of the proximal handle 8), leaving the guidewire (58) and anchor assembly (2, 4, 6) in place. The load assisting member (48) may be kept in position to assist with mechanical stabilization during this phase. Figure 1Q depicts further retraction of the needle member proximally into the proximal member (6) of the anchor assembly. At this stage, an expandable tract (32) has been carefully and safely created through the wall (18) of the vessel (16) in a configuration wherein, as described below, it preferably will self-seal when instrumentation is removed from the tract - and a guidewire (58) has been left in place through the expandable tract (32). The load assisting member (48) may be moved (92) back into a non-deployed position by applying or releasing a load proximally at the handle (8). Further movement (92) of the load assisting member (48) is illustrated in Figure 1S. With the load assisting member (48) either retracted fully into the body of the anchor assembly midportion (4), or left in a configuration wherein it will not appreciably cause additional resistance to proximal pullout of the assembly, the assembly may be pulled out (94), as shown in Figure 1T, leaving behind a configuration such as that shown in Figure 1U, wherein the guidewire (58) remains positioned through the expandable tract (32), and wherein the relatively small initial first passage (22) is closed, preferably by natural hemostasis due to the relatively small size of the first passage (22) that is required to deploy the subject instrumentation.

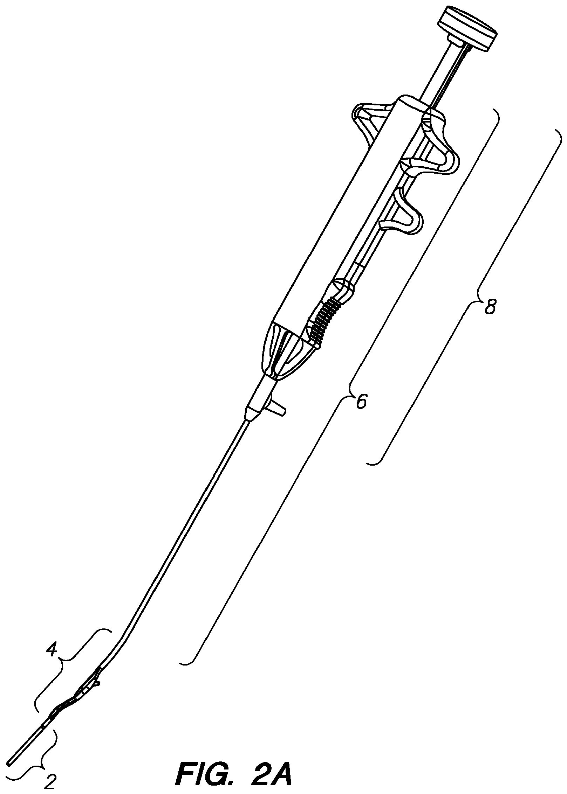

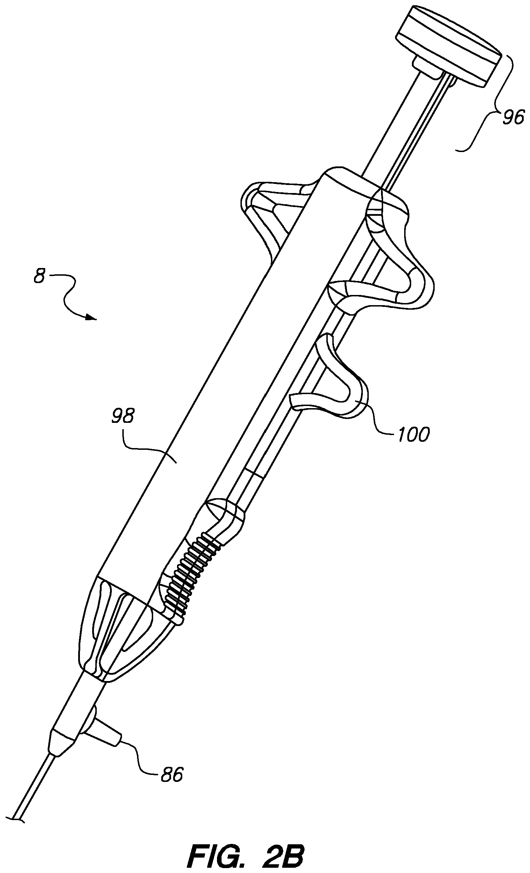

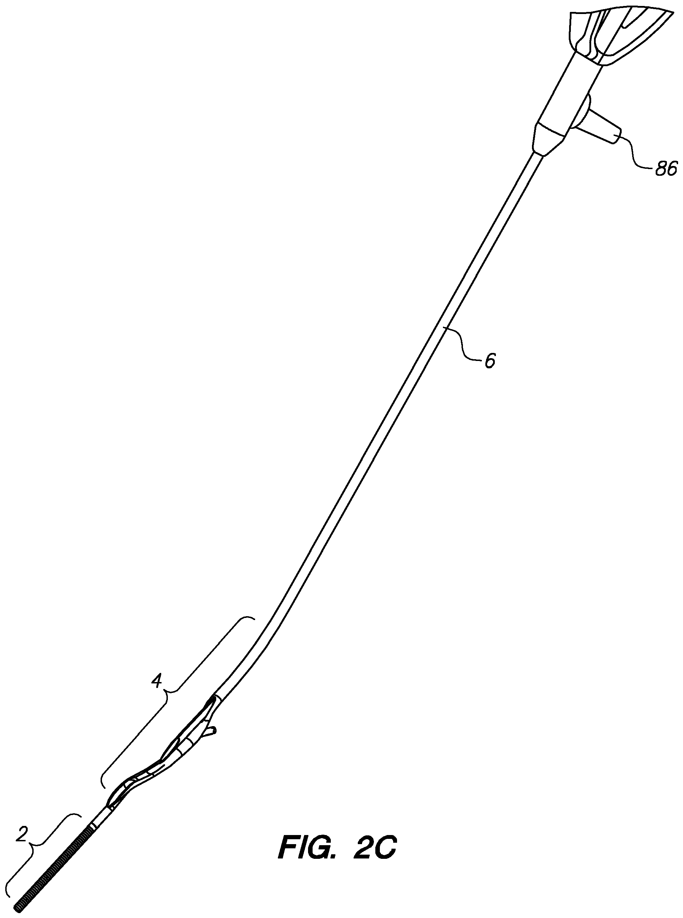

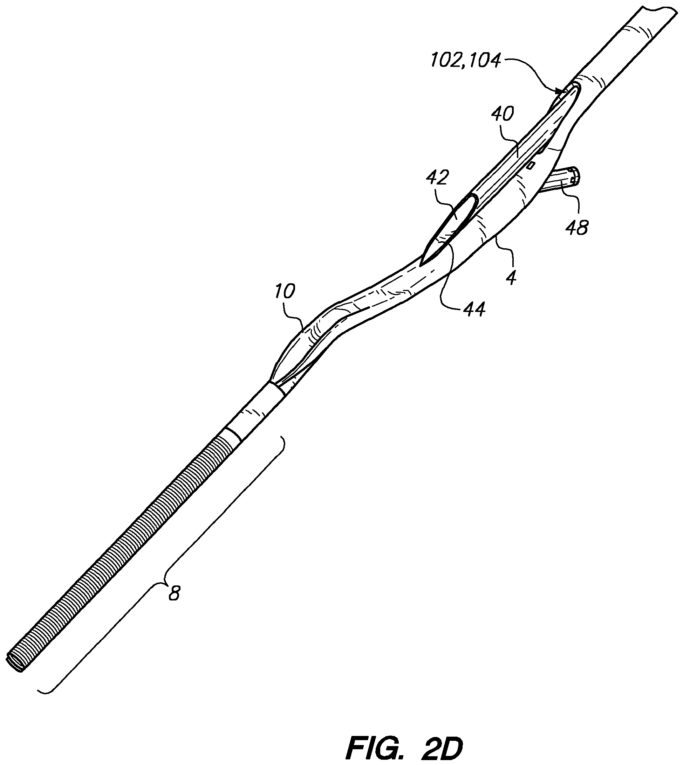

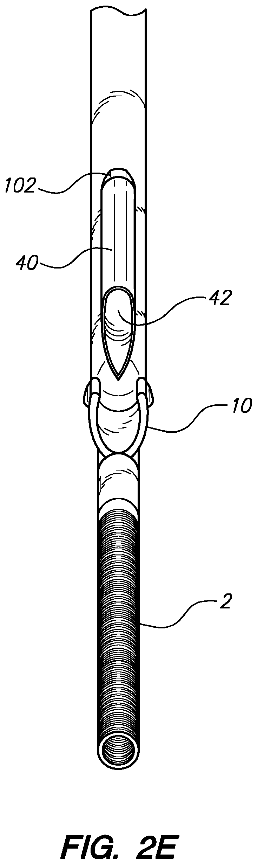

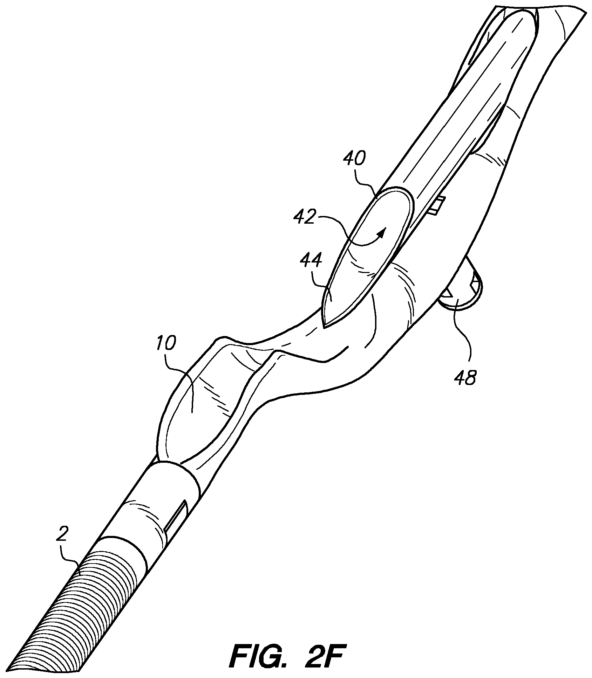

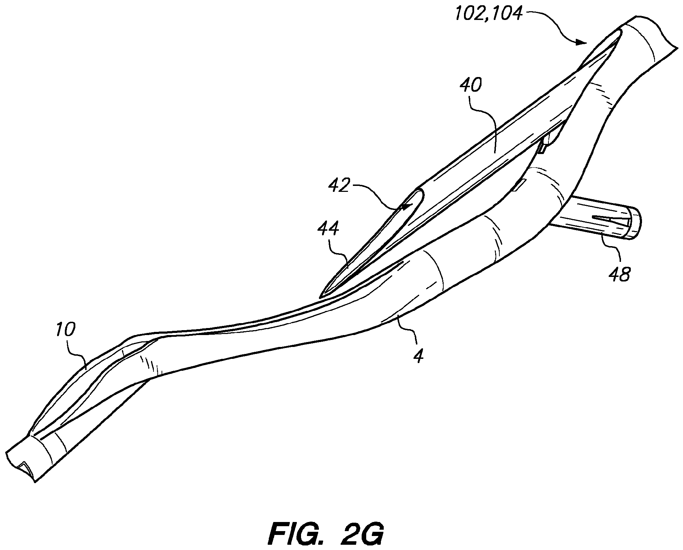

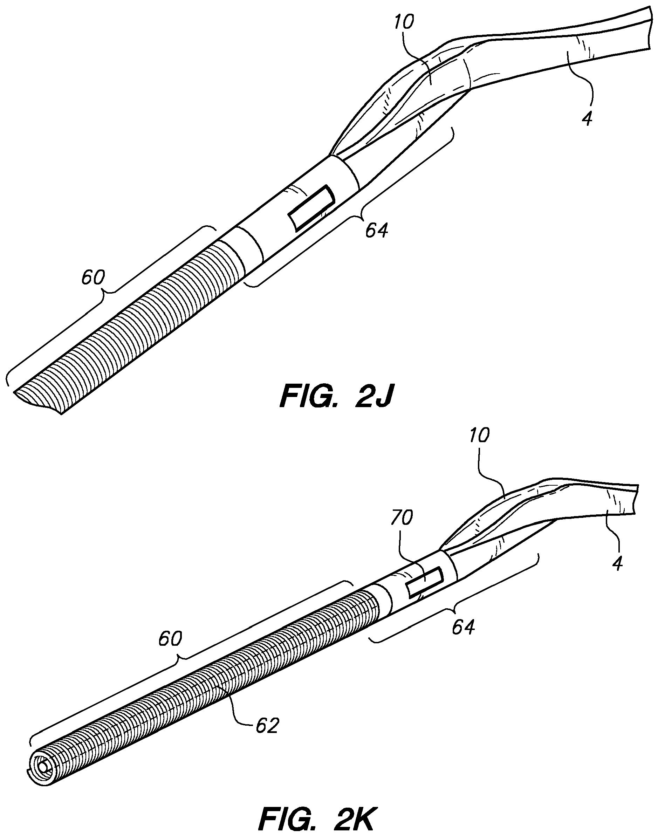

Referring to Figures 2A-2T, three dimensional illustrations are depicted to assist with visualizing various aspects of embodiments of the invention. Referring to Figure 2A, an assembly is depicted comprising a flexible distal anchor portion (2) coupled to a prebent anchor midportion (4), coupled to a proximal portion (6) comprising a handle assembly (8). Figure 2B shows a closer view of the handle assembly (8), featuring a needle plunger (96) movably coupled to a main housing (98), the needle plunger coupled to a needle such as those described above (element 40). A deployment member (100) also is movably coupled to the main housing (98), and is coupled, in one embodiment via a tension element, to a load assisting member (48) which may be controllably deployed away from a portion of an anchor assembly, as described above. Also shown in the close up view of Figure 2B is a proximal mark port (86) which may be utilized to assist and operator in positioning an anchoring assembly relative to a vascular lumen, also as described above. Figure 2C illustrates a close-up view of portions of an anchor assembly, including a flexible distal portion (2), a prebent midportion (4), here movably coupled to a hollow needle (40), and an elongate proximal portion (6). Figure 2D illustrates yet a closer view of portions of an anchor assembly and associated movable needle. Referring to Figure 2D, a flexible distal portion (2) may comprise a coiled wire construct. The distal aspect of the prebent midportion (4) comprises a saddle-shaped needle receiving structure (10), which, as described above, may be configured to provide a mechanically constraining geometry for a needle (40) to slide into. In one embodiment, about ½ of the outer surface of the associated needle (40) local to the receiving structure (10) may be physically constrained via a movable association with the receiving structure (10) geometry. The view of Figure 2D illustrates that in one embodiment, a needle (40) may be movably coupled through a port (104) and associated lumen (102) defined through a portion of the anchor assembly midportion (4) or proximal portion (6). Figure 2E depicts an alternate view of similar structures. Figure 2F depicts a further closer view of an anchor assembly and associated needle (40) with lumen (42) defined therethrough to accommodate a guidewire or other elongate structure. The needle (40) tip (44) may comprise various cutting geometries, such as a trocar tip geometry, chiseled tip geometry, or scoop-like geometry. An alternative perspective view is depicted in Figure 2G, which includes a close side-view of a load-assisting member (48) deployed away from the pre-bent anchor midportion (4) to which it is movably coupled.

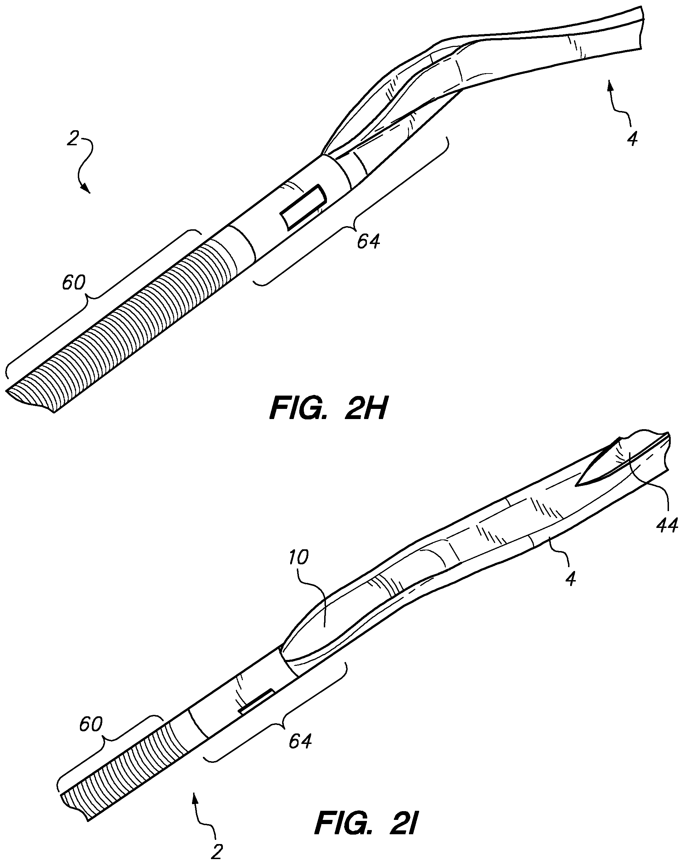

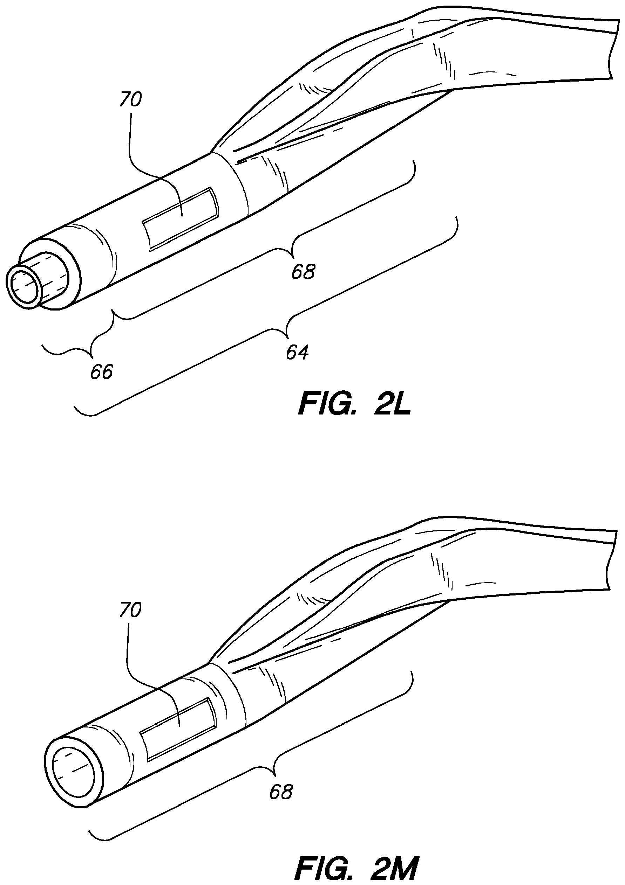

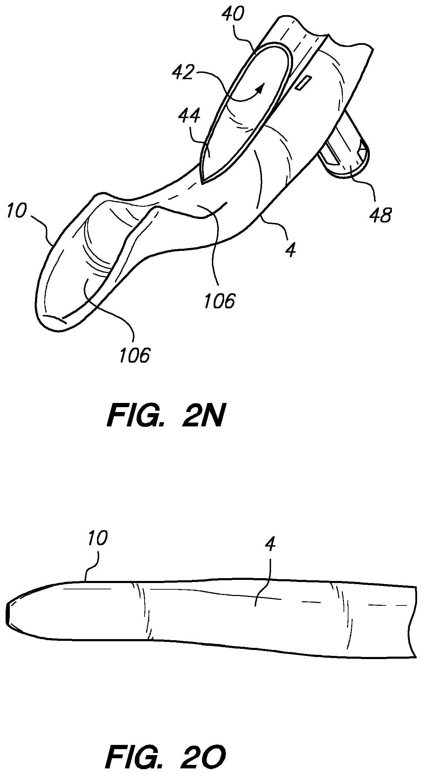

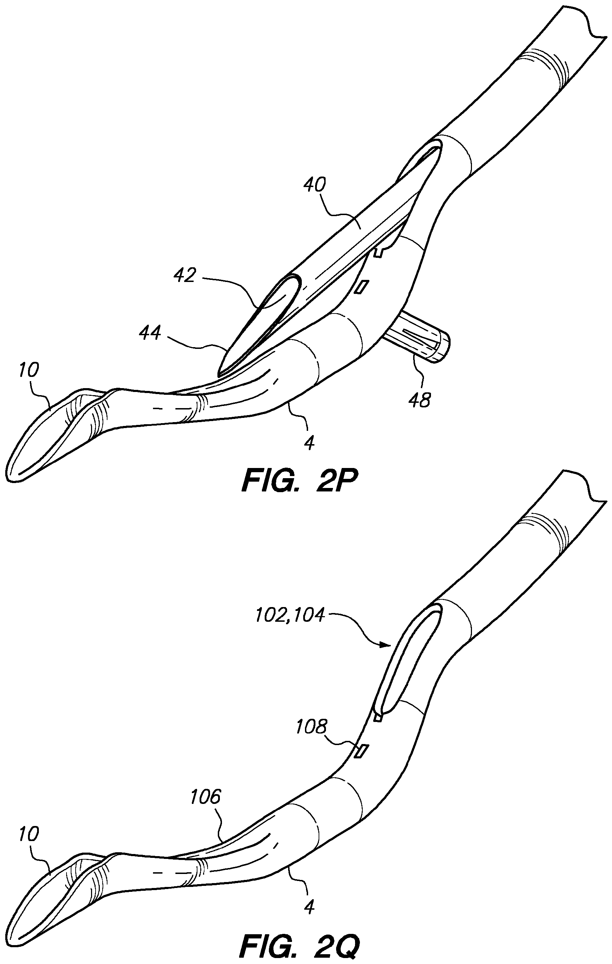

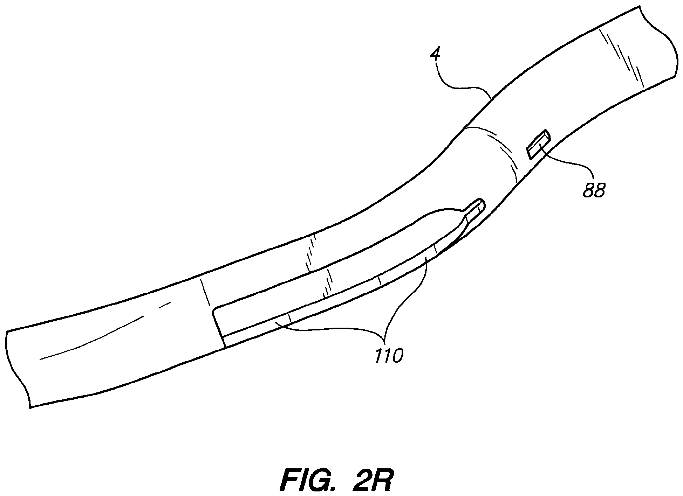

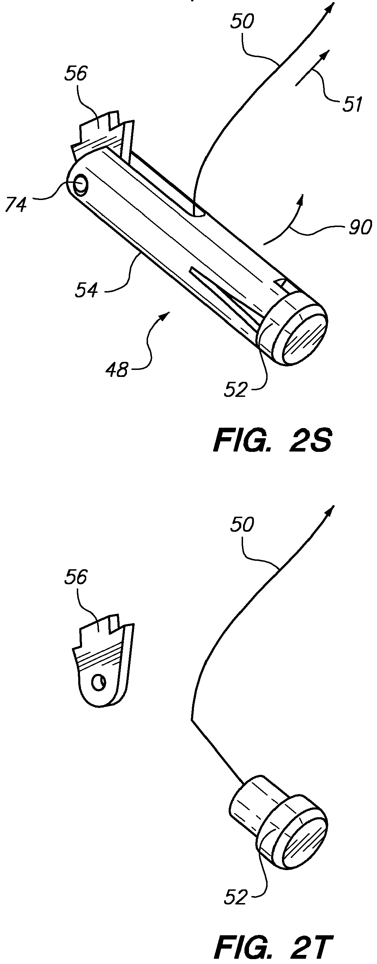

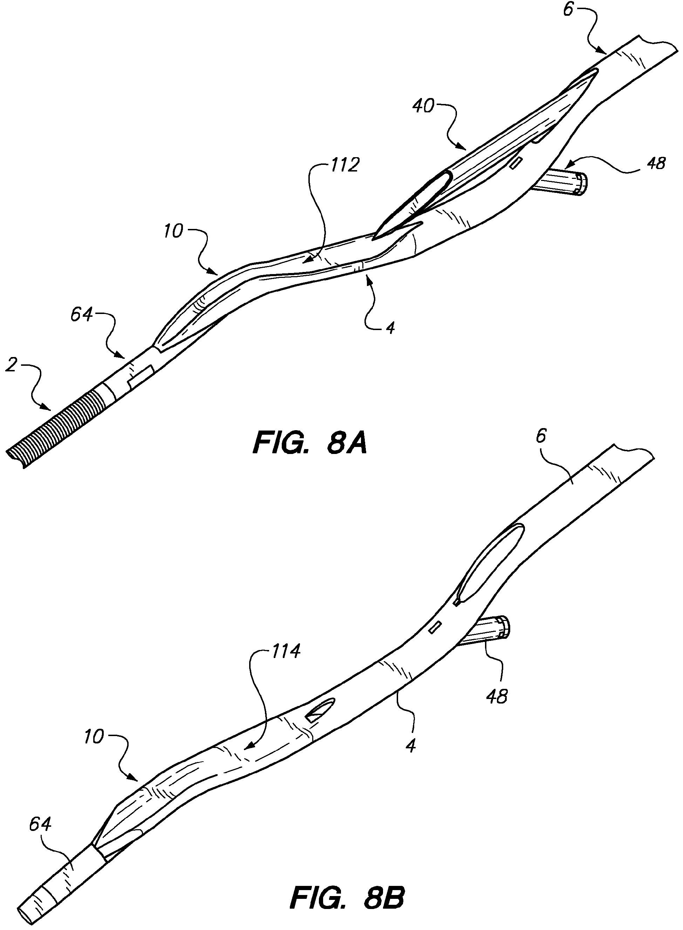

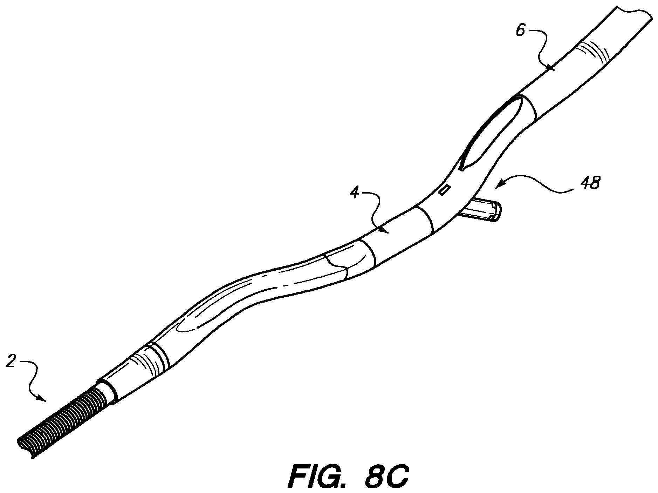

Referring to Figure 2H, a flexible distal anchor portion (2) may comprise a coiled wire portion (60) coupled to a saddle-shaped needle receiving structure (10) of a pre-bent anchor midportion (4) by a junction assembly (64) which, as shown in Figures 2L and 2M, may comprise a first coupling member (66) coupled to a coiled wire portion (60) and a structural core wire (element 62 in Figure 2K). The first coupling member (66) may be removably coupled to a second coupling member (68) using one or more latch features (70), such as the cantilevered tab (70) formed into a portion of the second coupling member (68) as shown in Figure 2M. Figures 2I and 2K shown differing perspective close-up views to illustrate the shape of the saddle-shaped needle receiving structure (10) formed into the distal end of the pre-bent anchor midportion (4), which may be welded or glued to the junction assembly (64). As described below in reference to Figure 8C, a junction alternatively may be formed as an integral portion of a pre-bent anchor midportion. Referring to Figure 2N, a close-up view of a saddle-shaped needle receiving structure (10) formed into the distal end of the pre-bent anchor midportion (4) shows a valley-like passageway (106), akin to the "spine" of a canoe, that is formed into the prebent anchor midportion (4) to assist in guiding and constraining the motion of the associated needle (40) tip (44). Figure 2O illustrates an alternate view of a saddle-shaped needle receiving structure (10) formed into the distal end of the pre-bent anchor midportion (4). Referring to Figure 2P, with the junction assembly (64) and flexible distal portion (2) hidden, a close-up orthogonal view highlights the saddle-shaped needle receiving structure (10) formed into the distal end of the pre-bent anchor midportion (4) and associated needle (40) which is movably coupled thereto. Also shown is the load assisting member (48) in a deployed out position. Figure 2Q illustrates a prebent anchor midportion (4) without an associated flexible distal portion (2) or movably coupled needle (40). A port (104) and lumen (102) are shown formed into the midportion (4) using techniques such as laser cutting, to accommodate a needle (40) as described above. A load assisting member hinge coupling slot (108) may be similarly formed. The valley (106) and saddle-like (10) geometries may be created by mechanically manipulating an otherwise straight or bent piece of tubing to yield a fairly atraumatic outer geometry. Alternatively, such features may be laser cut into a piece of tubing, and any sharp edges may be polished away and/or rolled over to provide for atraumatic tissue interfacing. Figure 2R shows an underside view of a similar structure with lasercut features for a load assisting member (48) to be moved into upon insertion or retraction of the pre-bent anchor midportion (4); another lasercut feature comprises a small cutout feature or aperture (88) created for a tensile element (50) to be passed through en route to coupling to a distal end of a load assisting member (48), as described below. Figure 2S illustrates a close-up view of a suitable load assisting member (48) with a coupling hinge member (56) that may be coupled to the pre-bent anchor midportion (4) at the coupling slot (108). A tubular body (54) coupled to a distal plug member (52) may be rotatably connected to the hinge member (56) with a pivot joint pin (74). Figure 2T illustrates a similar grouping of elements without the main tubular body (54) shown to illustrate coupling of a tension element (50) to a distal structure such as the distal plug member (52), with a configuration designed to cause rotation (90) of the load assisting member (48) about the pivot joint pin (74) axis under tension through the tension element (50). The load assisting member (48) and associated anchor midportion (4) may be intercoupled with a spring that is biased to maintain a closed configuration until a tensile load is applied in the tension member (50), after which deployment outward may occur and remain until the tensile load is released.

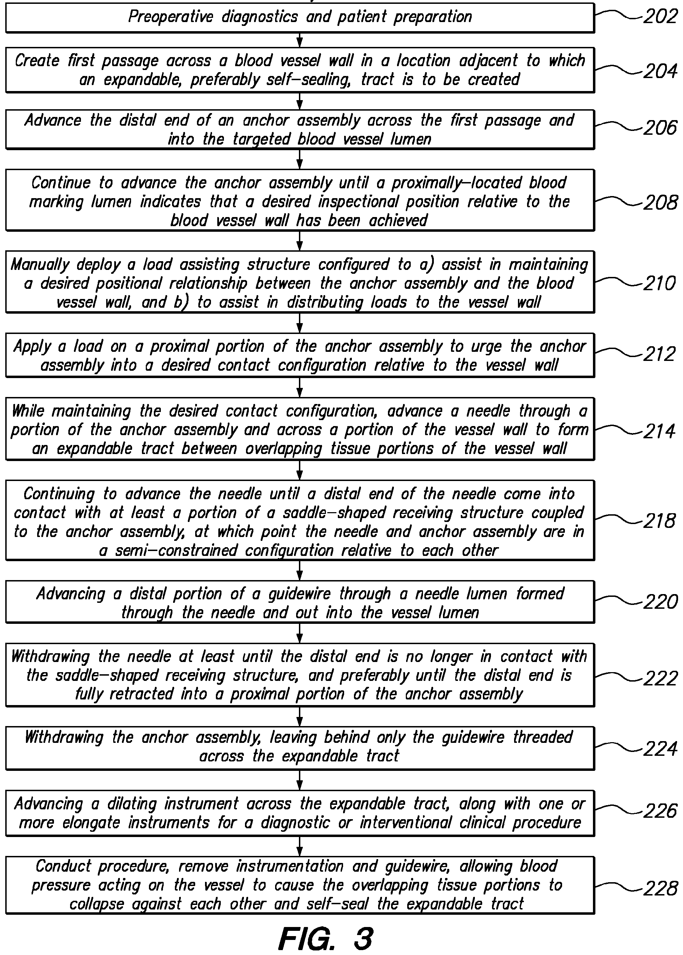

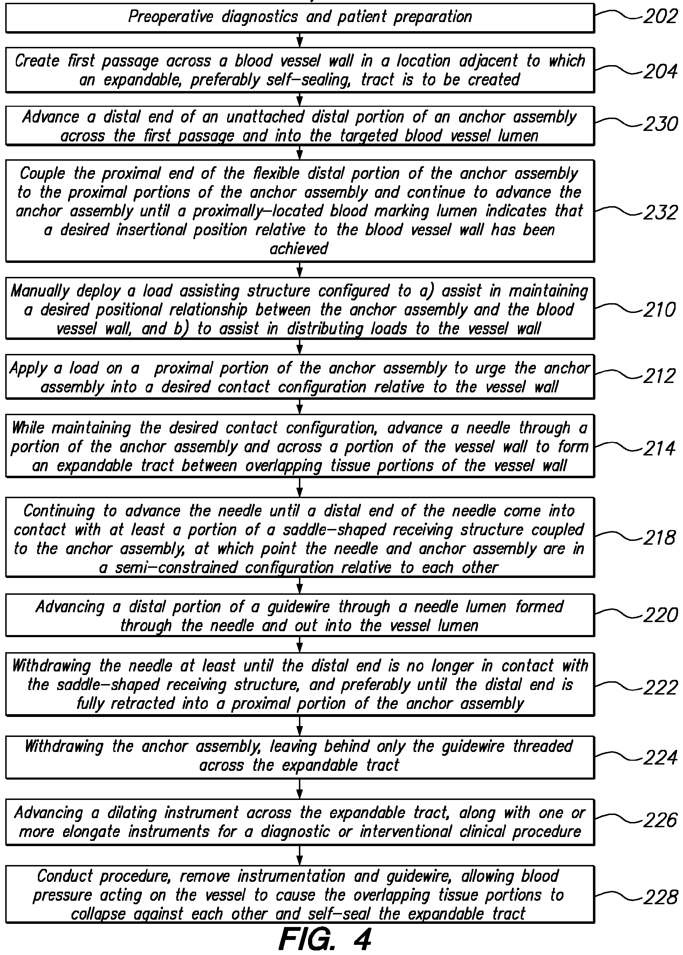

Referring to Figures 3 and 4, various embodiments of procedures utilizing the subject technology are illustrated. Referring to Figure 3, after diagnostics and patient preparation for a diagnostic, interventional, and/or surgical procedure (202), a first passage may be created across a wall of a blood vessel and adjacent skin and subdermal tissue adjacent a location wherein an expandable, preferably self-sealing tract is to be created and utilized in a surgical procedure (204). With the first passage created, a distal end of an anchor assembly (the assembly generally comprising a flexible distal portion, a pre-bent midportion, and a proximal portion for manipulation and needle coupling) may be advanced across the first passage and into the targeted blood vessel (206). Advancement may be continued until a desired insertion configuration is achieved; this may be determined utilizing a blood marking configuration with a distally located port fluidly coupled to a proximal blood marking port (208). A load-assisting structure may be deployed (210) and utilized in loading the anchor assembly into a desired contact configuration with the associated vessel wall (212). While maintaining this desired contact configuration, a needle may be advanced at a carefully controlled trajectory across the vessel wall to create an expandable tract between two overlapping portions of the tissue wall (214). The needle advancement may be continued until a needle distal end contacts at least a portion of a constraining saddle-shaped receiving structure of the anchor assembly (218). While keeping the needle in place, a guidewire or other flexible elongate member may be inserted through a working lumen defined through the needle (220). The needle may then be withdrawn (222), followed by withdrawal of the anchor assembly (224), leaving behind only the guidewire placed across the expandable tract. This this self-sealing expandable tract created, a diagnostic and/or surgical procedure may be conducted using the tract, for example, by inserting a dilating instrument, such as a tapered introducer catheter, across the tract, along with other pertinent instruments (226) and conducting the diagnostic and/or interventional procedure. With the procedure completed, the instrumentation, guidewire, and dilating instrument may be removed in various orders (228), with removal of the last allowing blood pressure acting on the vessel to cause the overlapping tissue portions to collapse against each other and "self seal" the expandable tract. For example, in one embodiment, the surgical instrumentation may first be removed, followed by the dilator, leaving only the guidewire to be finally removed for self-sealing. In another embodiment the dilator may be last removed. In another embodiment, all may be removed together to effect self sealing of the tract.

The embodiment of Figure 4 differs from that of Figure 3 in that the flexible distal end of the anchor assembly is inserted into the lumen unattached from the other portions of the anchor assembly (230), after which it is coupled with the remainder of the anchor assembly (232) with the distal portion in situ.

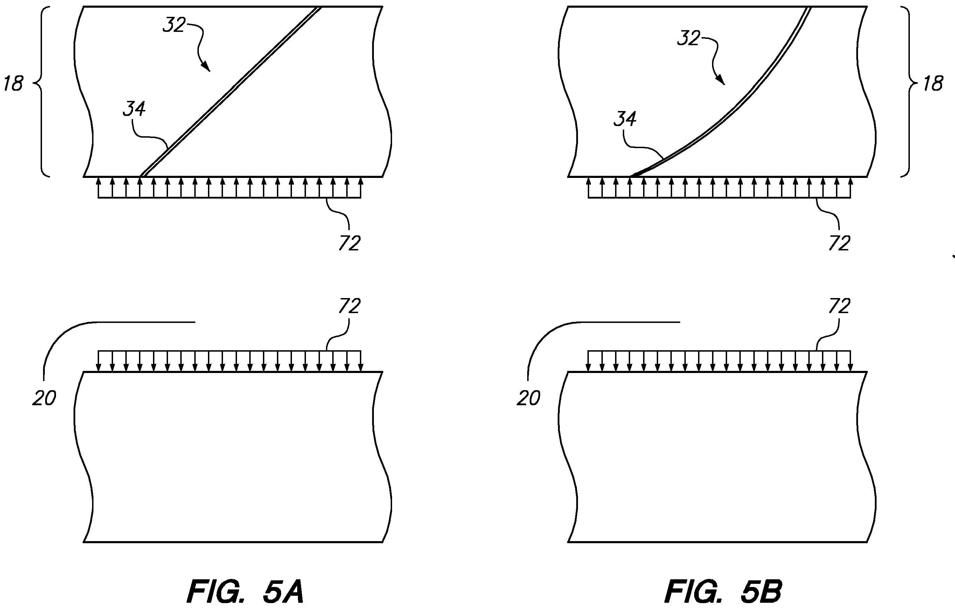

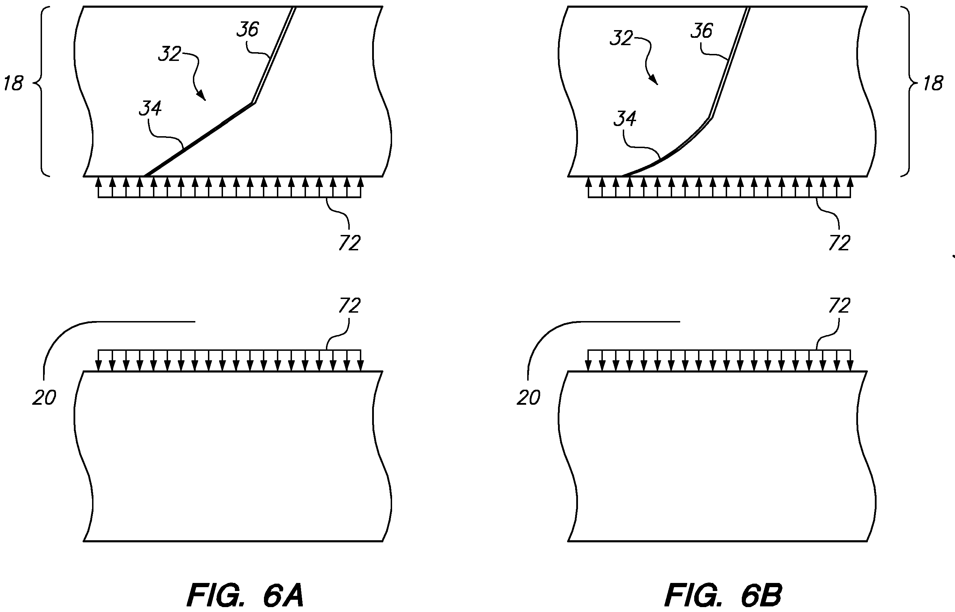

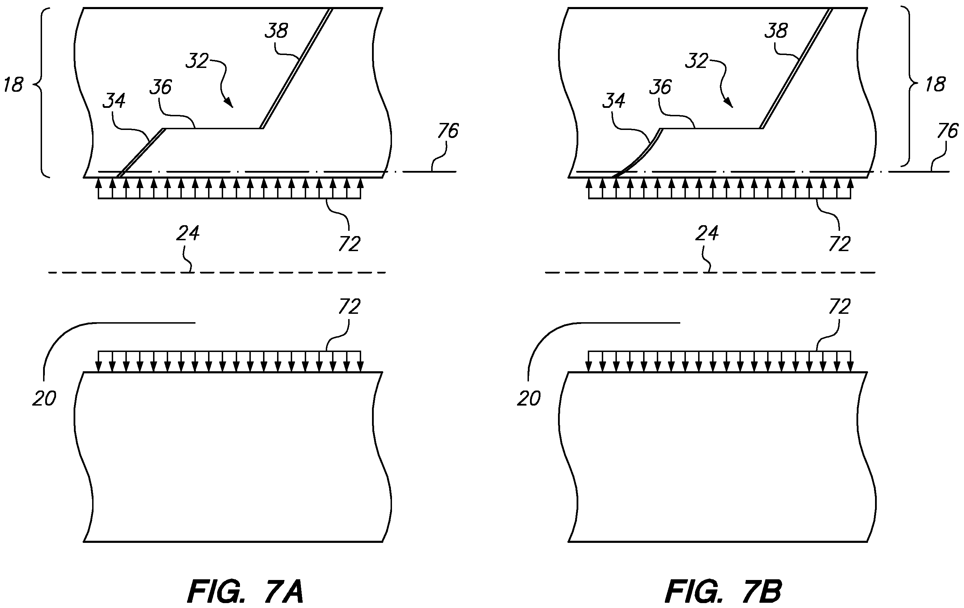

Referring to Figures 5A-7B, various illustrations of partial cross sections of vessel walls (18) are shown to depict various expandable tract (32) configurations that are within the scope of the present invention. Referring to Figure 5A, a single segment (34) expandable tract has been created that is substantially straight relative to the lumen (20) of the vessel. Pressure (72) from pressurized blood within the lumen will act to self-seal the overlapping tissue portions bisected by the angled expandable tract (32). Figure 5B features a single segment (34) self-sealing expandable tract (32) formed in an arcuate shape. Such an arcuate shape maybe created for an entire expandable tract or segment thereof using techniques such as a steerable needle, a needle with a predetermined insertion trajectory profile, and/or a needle with a cutting tip that results in an arcuate trajectory through tissue. Referring to Figures 6A, two segment (34, 36) expandable self-sealing tract configurations are depicted having straight and/or arcuate segments. Three segment (34, 36, 38) configurations of straight or nonstraight segments are shown in Figures 7A and 7B. To assist with the self-sealing action of the overlapping tissue portions bisected by the tract, the general trajectory of segments closer to the lumen (20) may be more closely parallel to the lumen longitudinal axis (shown as element 24 in Figure 7B) or an axis parallel to the nearby vessel wall (shown as element 76 in Figure 7B).

Referring to Figures 8A-8C, other embodiments of anchor assemblies are depicted with and without other associated elements, such as movably coupled needles. As shown in Figure 8A, an anchor assembly is depicted comprising a saddle-shaped distal aspect (10) of a pre-bent midportion (4), wherein a relatively large elongate portion (112) of the midportion (4) is formed into a "valley" or saddle-shaped proximal continuation of the more distal saddle-shaped feature (10). This proximal extension of the constraining geometry may be utilized to further guide a needle (40) as such needle is inserted to create an expandable tract. In the depicted embodiment, the proximal extension (112) of the constraining valley-like geometry is configured to contact, and thereby geometrically constrain from lateral movement, between about ¼ and about ½ of the cross sectional geometry of portions of the needle (40) which may be interfaced therewith. As with the saddle-shaped structure depicted above, for example, in Figure 2N, at least a portion of the saddle-shaped structure may be formed using laser cutting techniques to remove one or more portions of a tubelike structure, subsequent to which any edges left from cutting may be made relatively atraumatic by smoothing such edges or rolling the material local thereto. Referring to Figure 8B, another embodiment of an anchor assembly is depicted wherein the distal saddle-shaped structure (10) of the pre-bent midportion (4) is formed by deformation of a tubular element, thereby producing a pre-bent midportion that is double-walled throughout its length (with the exception of cutouts for elements such as a needle to pass through), and that has a distal aspect (10) with an essentially uninterrupted and rounded surface, which may be desirable from an atraumatic insertion perspective. Figure 8C depicts an embodiment similar to that depicted in Figure 8B, with the exception that the junction member (64) shown in Figure 8B, which may be coupled to the pre-bent midportion (4) during device assembly, is replaced in the embodiment of Figure 8C with an all-in-one configuration, wherein the distal aspect of the pre-bent midportion (4) of the embodiment of Figure 8C comprises a junction fitting and saddle-shaped, or valley-shaped, constraining structure (116) that are formed from the same piece of material (118), such as by controlled deformation and/or machining of a tubular structure. Such a configuration may be preferred for ease of manufacture and/or enhanced or more homogeneous device bending modulus reasons.

Various exemplary embodiments of the invention are described herein. Reference is made to these examples in a non-limiting sense. They are provided to illustrate more broadly applicable aspects of the invention. Various changes may be made to the invention described and equivalents may be substituted without departing from the scope of the invention.

Any of the devices described for carrying out the subject diagnostic or interventional procedures may be provided in packaged combination for use in executing such interventions. These supply "kits" may further include instructions for use and be packaged in sterile trays or containers as commonly employed for such purposes.

Methods that may be performed using the subject devices. The methods may comprise the act of providing such a suitable device. Such provision may be performed by the end user. In other words, the "providing" act merely requires the end user obtain, access, approach, position, set-up, activate, power-up or otherwise act to provide the requisite device in the subject method. Methods recited herein may be carried out in any order of the recited events which is logically possible, as well as in the recited order of events.

Exemplary aspects of the invention, together with details regarding material selection and manufacture have been set forth above.

Also, it is contemplated that any optional feature of the inventive variations described may be set forth and claimed independently, or in combination with any one or more of the features described herein. Reference to a singular item, includes the possibility that there are plural of the same items present. More specifically, as used herein and in claims associated hereto, the singular forms "a," "an," "said," and "the" include plural referents unless the specifically stated otherwise. In other words, use of the articles allow for "at least one" of the subject item in the description above as well as claims associated with this disclosure. It is further noted that such claims may be drafted to exclude any optional element. As such, this statement is intended to serve as antecedent basis for use of such exclusive terminology as "solely," "only" and the like in connection with the recitation of claim elements, or use of a "negative" limitation.

Without the use of such exclusive terminology, the term "comprising" in claims associated with this disclosure shall allow for the inclusion of any additional element--irrespective of whether a given number of elements are enumerated in such claims, or the addition of a feature could be regarded as transforming the nature of an element set forth in such claims. Except as specifically defined herein, all technical and scientific terms used herein are to be given as broad a commonly understood meaning as possible while maintaining claim validity.

The breadth of the present invention is not to be limited to the examples provided and/or the subject specification, but rather only by the scope of claim language associated with this disclosure.

## Claims

1. A device for forming an expandable tract (32) across a wall (18) of a blood vessel (16), comprising:
a needle (40);
an anchor assembly comprising a proximal portion (6) having a handle (8), a flexible distal portion (2), and a pre-bent midportion (4) intercoupled between the proximal (6) and distal portions, wherein the needle (40) is configured to be inserted through a portion of the proximal portion (6);
wherein at least a distal tip of the flexible distal portion (2) is configured to be placed within a lumen (20) of the blood vessel (16) through a first passage (22) created across the wall (18) with a sharpened member at a first angle relative to a lumen longitudinal axis defined by the lumen (20) of the blood vessel (16) in the region adjacent the first passage (22); and
**characterized in that**
said pre-bent midportion (4) comprises an at least partially encapsulating saddle-shaped needle receiving structure configured to receive and support the needle (40),
wherein the needle (40) and anchor assembly are configured such that upon applying a force to the anchor assembly to position an adjacent portion of the blood vessel wall (18) into a desired contact configuration relative to the anchor assembly, the needle (40) is operatively coupled to the anchor assembly such that it may be advanced across the wall of the blood vessel (18) and into contact with said at least partially encapsulating saddle-shaped needle receiving structure, thereby creating an expandable tract (32) between overlapping tissue portions of the vessel wall (18).

2. The device of claim 1, wherein the proximal portion (6) comprises an elongate tubular member through which the needle (40) may be slidably coupled.

3. The device of claim 1, wherein the needle (40) comprises a hollow needle defining a working lumen therethrough.

4. The device of claim 3, wherein the needle (40) comprises a trocar or chisel tip geometry.

5. The device of claim 1, wherein the anchor assembly is configured to direct the needle (40) in a substantially straight trajectory across the wall (18) of the blood vessel (16) and into contact with the at least partially encapsulating saddle-shaped needle receiving structure.

6. The device of claim 1, wherein the anchor assembly and needle (40) are configured to direct the needle (40) in an arcuate trajectory across the wall (18) of the blood vessel (16) and into contact with the at least partially encapsulating saddle-shaped needle receiving structure.

7. The device of claim 1, wherein the anchor assembly and needle (40) are configured to direct the needle (40) in a two part trajectory across the wall (18) of the blood vessel (16) and into contact with the at least partially encapsulating saddle-shaped needle receiving structure, wherein a distal portion of the needle trajectory forms a distal portion of the expandable tract (32) that is angled more steeply relative to the lumen longitudinal axis than is a proximal portion of the expandable tract (32).

8. The device of claim 7, wherein the anchor assembly and needle (40) are configured to direct the needle (40) in a two part trajectory across the wall (18) of the blood vessel (16) and into contact with the at least partially encapsulating saddle-shaped needle receiving structure such that proximal portion of the expandable tract (32) is substantially parallel with the lumen longitudinal axis.

9. The device of claim 1, further comprising a load assisting member (48) movably coupled to the anchor assembly configured to be controllably extended from the anchor assembly before applying the force to the anchor assembly.

10. The device of claim 9, wherein the load assisting member (48) is controllably rotatable about a pivot point relative to the anchor assembly.

11. The device of claim 9, wherein the load assisting member (48) is controllably insertable outward from an outer surface of the anchor assembly along a substantially straight axial pathway relative to the anchor assembly.

12. The device of claim 9, wherein the load assisting member (48) is controllably insertable outward from an outer surface of the anchor assembly along an arcuate pathway relative to the anchor assembly.

13. The device of claim 9, further comprising a proximal load applying member operatively coupled to the handle (8) and configured to transfer a load from a proximal portion (6) of the anchor assembly to one or more members coupled to the load assisting structure.

14. The device of claim 1, further comprising a guidewire (58) insertable through the expandable tract (32).

15. The device of claim 14, wherein the guidewire (58) is inserted through at least a portion of the needle (40).

16. The device of claim 1, further comprising a dilating instrument insertable across the expandable tract (32).

17. The device of claim 1, wherein the flexible distal portion (2) of the anchor assembly comprises a wire formed into a longitudinal coil.

18. The device of claim 17, further comprising an elongate structural core wire positioned through a lumen defined through the longitudinal coil.

19. The device of claim 18, wherein the elongate structure core wire comprises a noncircular cross sectional geometry configured to impart nonhomogeneous bending characteristics upon the flexible distal portion of the anchor assembly.

20. The device of claim 19, wherein the noncircular cross sectional geometry comprises a rectangular cross sectional shape.

21. The device of claim 1, wherein a proximal end of the flexible distal portion (2) of the anchor assembly is removably coupled to a distal end of the pre-bent midportion (4).

22. The device of claim 21, wherein the proximal end of the flexible distal portion (2) of the anchor assembly is removably coupled to the distal end of the prebent midportion (4) using a mechanical latch fitting.

23. The device of claim 1, wherein the anchor assembly and needle (40) are configured to create the expandable tract (32) to have a geometry relative to the wall of the blood vessel such that upon withdrawal of structures from the expandable tract (32), blood pressure acting on the vessel wall causes the overlapping tissue portions to collapse against each other and self-seal the expandable tract (32).

24. The device of claim 1, wherein the at least partially encapsulating saddle-shaped needle receiving structure comprises a laser-cut tubular member portion.

25. The device of claim 1, wherein the at least partially encapsulating saddle-shaped needle receiving structure comprises a mechanically-formed tubular member portion.

26. The device of claim 1, wherein the at least partially encapsulating saddle-shaped needle receiving structure has a geometry configured to encapsulate about 1/2 of the surface geometry of a distal tip of the needle when interfaced with the needle (40).

## Patentansprüche

1. Vorrichtung zur Bildung eines aufweitbaren Traktes (32) über eine Wand (18) eines Blutgefäßes (16), die Folgendes umfasst:
eine Nadel (40);
eine Ankeranordnung, die einen proximalen Abschnitt (6) mit einem Griff (8), einen flexiblen distalen Abschnitt (2) und einen vorgebogenen Mittelabschnitt (4) umfasst, der zwischen den proximalen (6) und dem distalen Abschnitt gekoppelt ist, wobei die Nadel (40) so ausgelegt ist, dass sie durch einen Abschnitt des proximalen Abschnitts (6) eingeführt werden kann;
wobei mindestens eine distale Spitze des flexiblen distalen Abschnitts (2) so konfiguriert ist, dass sie in einem Lumen (20) des Blutgefäßes (16) durch einen ersten Durchgang (22), der quer zur Wand (18) mit einem geschärften Element in einem ersten Winkel relativ zu einer Lumenlängsachse erzeugt wird, die durch das Lumen (20) des Blutgefäßes (16) in dem Bereich neben dem ersten Durchgang (22) definiert ist, angeordnet werden kann; und
**dadurch gekennzeichnet, dass**
der vorgebogene Mittelabschnitt (4) eine zumindest teilweise umhüllende sattelförmige Nadelaufnahmestruktur umfasst, die so konfiguriert ist, dass sie die Nadel (40) aufnimmt und trägt,
wobei die Nadel (40) und die Ankeranordnung so konfiguriert sind, dass beim Aufbringen einer Kraft auf die Ankeranordnung, um einen benachbarten Abschnitt der Blutgefäßwand (18) in eine gewünschte Kontaktkonfiguration relativ zur Ankeranordnung zu positionieren, die Nadel (40) funktionsfähig mit der Ankeranordnung gekoppelt ist, so dass sie über die Wand des Blutgefäßes (18) und in Kontakt mit der zumindest teilweise einkapselnden sattelförmigen Nadelaufnahmestruktur vorgeschoben werden kann, wodurch ein aufweitbarer Trakt (32) zwischen überlappenden Gewebeabschnitten der Gefäßwand (18) erzeugt wird.

2. Vorrichtung nach Anspruch 1, wobei der proximale Abschnitt (6) ein längliches röhrenförmiges Element umfasst, durch das die Nadel (40) gleitend gekoppelt werden kann.

3. Vorrichtung nach Anspruch 1, wobei die Nadel (40) eine Hohlnadel umfasst, die ein Arbeitslumen durch sie hindurch definiert.

4. Vorrichtung nach Anspruch 3, wobei die Nadel (40) eine Trokar- oder Meißelspitzengeometrie aufweist.

5. Vorrichtung nach Anspruch 1, wobei die Ankeranordnung so konfiguriert ist, dass sie die Nadel (40) in einer im Wesentlichen geraden Bahn über die Wand (18) des Blutgefäßes (16) und in Kontakt mit der zumindest teilweise verkapselnden sattelförmigen Nadelaufnahmekonstruktion führt.

6. Vorrichtung nach Anspruch 1, bei der die Ankeranordnung und die Nadel (40) so ausgelegt sind, dass sie die Nadel (40) in einer bogenförmigen Bahn über die Wand (18) des Blutgefäßes (16) und in Kontakt mit der zumindest teilweise verkapselnden sattelförmigen Nadelaufnahmestruktur führen.

7. Vorrichtung nach Anspruch 1, wobei die Ankeranordnung und die Nadel (40) so konfiguriert sind, dass sie die Nadel (40) in einer zweiteiligen Bahn quer über die Wand (18) des Blutgefäßes (16) und in Kontakt mit der zumindest teilweise verkapselnden sattelförmigen Nadelaufnahmestruktur führen, wobei ein distaler Abschnitt der Nadelbahn einen distalen Abschnitt des aufweitbaren Traktes (32) bildet, der relativ zur Lumenlängsachse steiler abgewinkelt ist als ein proximaler Abschnitt des aufweitbaren Traktes (32).

8. Vorrichtung nach Anspruch 7, bei der die Ankeranordnung und die Nadel (40) so konfiguriert sind, dass sie die Nadel (40) in einer zweiteiligen Bahn über die Wand (18) des Blutgefäßes (16) und in Kontakt mit der zumindest teilweise verkapselnden sattelförmigen Nadelaufnahmestruktur führen, so dass der proximale Teil des aufweitbaren Trakts (32) im Wesentlichen parallel zur Lumenlängsachse verläuft.

9. Vorrichtung nach Anspruch 1, die ferner ein beweglich mit der Ankeranordnung gekoppeltes Lastunterstützungselement (48) umfasst, das so ausgelegt ist, dass es vor dem Aufbringen der Kraft auf die Ankeranordnung steuerbar aus der Ankeranordnung ausgefahren werden kann.

10. Vorrichtung nach Anspruch 9, bei der das Lastunterstützungselement (48) steuerbar um einen Drehpunkt relativ zur Ankeranordnung drehbar ist.

11. Vorrichtung nach Anspruch 9, wobei das Lastunterstützungselement (48) von einer Außenfläche der Ankeranordnung entlang einer im Wesentlichen geraden axialen Bahn relativ zur Ankeranordnung steuerbar nach außen einsetzbar ist.

12. Vorrichtung nach Anspruch 9, wobei das Lastunterstützungselement (48) von einer Außenfläche der Ankeranordnung entlang einer bogenförmigen Bahn relativ zur Ankeranordnung steuerbar nach außen einsetzbar ist.

13. Vorrichtung nach Anspruch 9, die ferner ein proximales Lastaufbringungselement umfasst, das funktionsfähig mit dem Griff (8) gekoppelt und so ausgelegt ist, dass es eine Last von einem proximalen Abschnitt (6) der Ankeranordnung auf ein oder mehrere Elemente überträgt, die mit der Lastunterstützungsstruktur gekoppelt sind.

14. Vorrichtung nach Anspruch 1, die ferner einen Führungsdraht (58) umfasst, der durch den aufweitbaren Trakt (32) eingeführt werden kann.

15. Vorrichtung nach Anspruch 14, bei der der Führungsdraht (58) durch mindestens einen Teil der Nadel (40) eingeführt wird.

16. Vorrichtung nach Anspruch 1, die ferner ein Dilatationsinstrument umfasst, das über den aufweitbaren Trakt eingeführt werden kann (32).

17. Vorrichtung nach Anspruch 1, wobei der flexible distale Teil (2) der Ankeranordnung einen zu einer Längsspule geformten Draht umfasst.

18. Vorrichtung nach Anspruch 17, die ferner einen länglich strukturierten Kerndraht umfasst, der durch ein durch die Längsspule definiertes Lumen positioniert ist.

19. Vorrichtung nach Anspruch 18, bei der der länglich strukturierte Kerndraht eine nicht kreisförmige Querschnittsgeometrie aufweist, die so ausgelegt ist, dass sie dem flexiblen distalen Teil der Ankeranordnung inhomogene Biegeeigenschaften verleiht.

20. Vorrichtung nach Anspruch 19, bei der die nichtkreisförmige Querschnittsgeometrie eine rechteckige Querschnittsform aufweist.

21. Vorrichtung nach Anspruch 1, bei der ein proximales Ende des flexiblen distalen Abschnitts (2) der Ankeranordnung abnehmbar mit einem distalen Ende des vorgebogenen Mittelabschnitts (4) verbunden ist.

22. Vorrichtung nach Anspruch 21, bei der das proximale Ende des flexiblen distalen Teils (2) der Ankeranordnung mit dem distalen Ende des vorgebogenen Mittelteils (4) unter Verwendung einer mechanischen Verriegelungspassung lösbar gekoppelt ist.

23. Vorrichtung nach Anspruch 1, wobei die Ankeranordnung und die Nadel (40) so konfiguriert sind, dass sie den aufweitbaren Trakt (32) so gestalten, dass er eine solche Geometrie relativ zur Wand des Blutgefäßes aufweist, dass beim Zurückziehen von Strukturen aus dem aufweitbaren Trakt (32) der auf die Gefäßwand wirkende Blutdruck bewirkt, dass die überlappenden Gewebeteile gegeneinander zusammenfallen und den aufweitbaren Trakt (32) selbsttätig abdichten.

24. Vorrichtung nach Anspruch 1, bei der die zumindest teilweise umhüllende sattelförmige Nadelaufnahmekonstruktion einen lasergeschnittenen rohrförmigen Elementabschnitt aufweist.

25. Vorrichtung nach Anspruch 1, bei der die zumindest teilweise umhüllende sattelförmige Nadelaufnahmekonstruktion einen mechanisch geformten rohrförmigen Elementabschnitt aufweist.

26. Vorrichtung nach Anspruch 1, bei der die zumindest teilweise umhüllende sattelförmige Nadelaufnahmestruktur eine Geometrie aufweist, die so ausgelegt ist, dass sie etwa 1/2 der Oberflächengeometrie einer distalen Spitze der Nadel verkapselt, wenn sie mit der Nadel (40) verbunden ist.

## Revendications

1. Dispositif destiné à obtenir une voie (32) apte à s'élargir à travers une paroi (18) d'un vaisseau sanguin (16), comprenant :
une aiguille (40) ;
un assemblage d'ancrage comprenant une portion proximale (6) possédant une poignée (8), une portion distale flexible (2) et une portion médiane (4) incurvée de manière préalable qui permet de coupler de manière réciproque la portion proximale (6) et la portion distale ; dans lequel l'aiguille (40) est configurée pour venir s'insérer à travers une partie de la portion proximale (6) ;
dans lequel au moins une extrémité distale de la portion distale flexible (2) est configurée pour venir se placer au sein d'une lumière (20) du vaisseau sanguin (16) à travers un premier passage (22) créé à travers la paroi (18) comprenant un élément pointu qui forme un premier angle par rapport à un axe longitudinal de la lumière, défini par la lumière (20) du vaisseau sanguin (16) dans la zone adjacente au premier passage (22) ; et
**caractérisé en ce que**
ladite portion médiane (4) incurvée de manière préalable comprend une structure de réception d'aiguille possédant une configuration en forme de selle, qui procure un encapsulage au moins partiel, configurée pour recevoir et pour supporter l'aiguille (40) ;
dans lequel l'aiguille (40) et l'assemblage d'ancrage sont configurés d'une manière telle que lorsqu'on exerce une force sur l'assemblage d'ancrage dans le but de positionner une portion adjacente de la paroi (18) du vaisseau sanguin dans une configuration de contact désirée par rapport à l'assemblage d'ancrage, l'aiguille (40) est couplée de manière opérationnelle à l'assemblage d'ancrage d'une manière telle qu'elle est capable de progresser à travers la paroi du vaisseau sanguin (18) et d'entrer en contact avec ladite structure de réception d'aiguille possédant une configuration en forme de selle, qui procure un encapsulage au moins partiel, dans le but d'obtenir de cette manière une voie (32) apte à s'élargir entre des portions de tissu chevauchantes de la paroi de vaisseau (18).

2. Dispositif selon la revendication 1, dans lequel la portion proximale (6) comprend un élément tubulaire allongé par l'intermédiaire duquel l'aiguille (40) peut être couplée par glissement.

3. Dispositif selon la revendication 1, dans lequel l'aiguille (40) comprend une aiguille creuse définissant une lumière de travail à travers elle.

4. Dispositif selon la revendication 3, dans lequel l'aiguille (40) comprend une géométrie en forme de trocart ou une géométrie en forme de pointe biseautée.

5. Dispositif selon la revendication 1, dans lequel l'assemblage d'ancrage est configuré pour orienter l'aiguille (40) dans une trajectoire essentiellement rectiligne travers la paroi (18) du vaisseau sanguin (16) dans le but de la mettre en contact avec ladite structure de réception d'aiguille possédant une configuration en forme de selle, qui procure un encapsulage au moins partiel.

6. Dispositif selon la revendication 1, dans lequel l'assemblage d'ancrage et l'aiguille (40) sont configurés pour orienter l'aiguille (40) dans une trajectoire arquée à travers la paroi (18) du vaisseau sanguin (16) dans le but de la mettre en contact avec ladite structure de réception d'aiguille possédant une configuration en forme de selle, qui procure un encapsulage au moins partiel.

7. Dispositif selon la revendication 1, dans lequel l'assemblage d'ancrage et l'aiguille (40) sont configurés pour orienter l'aiguille (40) dans une trajectoire en deux parties à travers la paroi (18) du vaisseau sanguin (16) dans le but de la mettre en contact avec ladite structure de réception d'aiguille possédant une configuration en forme de selle, qui procure un encapsulage au moins partiel ; dans lequel une portion distale de la trajectoire de l'aiguille représente une portion distale de la voie (32) apte à s'élargir, qui forme, par rapport à l'axe longitudinal de la lumière, un angle plus incliné que l'angle formé par une portion proximale de la voie (32) apte à s'élargir.

8. Dispositif selon la revendication 7, dans lequel l'assemblage d'ancrage et l'aiguille (40) sont configurés pour orienter l'aiguille (40) dans une trajectoire en deux parties à travers la paroi (18) du vaisseau sanguin (16) dans le but de la mettre en contact avec ladite structure de réception d'aiguille possédant une configuration en forme de selle, d'une manière telle que la portion proximale de la voie (32) apte à s'élargir est essentiellement parallèle à l'axe longitudinal de la lumière.

9. Dispositif selon la revendication 1, comprenant en outre un élément de renfort de charge (48) qui est couplé en mobilité à l'assemblage d'ancrage configuré pour s'étendre de manière réglable à partir de l'assemblage d'ancrage avant d'exercer la force sur l'assemblage d'ancrage.

10. Dispositif selon la revendication 9, dans lequel l'élément de renfort de charge (48) est à même d'effectuer des rotations de manière réglable autour d'un point faisant office de pivot par rapport à l'assemblage d'ancrage.

11. Dispositif selon la revendication 9, dans lequel l'élément de renfort de charge (48) peut venir s'insérer de manière réglable à l'extérieur par rapport à une surface externe de l'assemblage d'ancrage le long d'une voie axiale essentiellement rectiligne par rapport à l'assemblage d'ancrage.

12. Dispositif selon la revendication 9, dans lequel l'élément de renfort de charge (48) peut venir s'insérer de manière réglable à l'extérieur par rapport à une surface externe de l'assemblage d'ancrage le long d'une voie arquée par rapport à l'assemblage d'ancrage.

13. Dispositif selon la revendication 9, comprenant en outre un élément proximal exerçant une charge, couplé de manière opérationnelle à la poignée (8) et configuré pour transférer une charge à partir d'une portion proximale (6) de l'assemblage d'ancrage à un ou plusieurs éléments couplés à la structure de renfort de charge.

14. Dispositif selon la revendication 1, comprenant en outre un fil de guidage (58) qui peut venir s'insérer à travers la voie (32) apte à s'élargir.

15. Dispositif selon la revendication 14, dans lequel le fil de guidage (58) est inséré à travers au moins une portion de l'aiguille (40).

16. Dispositif selon la revendication 1, comprenant en outre un instrument de dilatation qui peut venir s'insérer à travers la voie (32) apte à s'élargir.

17. Dispositif selon la revendication 1, dans lequel la portion distale flexible (2) de l'assemblage d'ancrage comprend un fil métallique façonné pour obtenir un serpentin longitudinal.

18. Dispositif selon la revendication 17, comprenant en outre un fil métallique de structure allongée faisant office de partie centrale, disposé à travers une lumière définie à travers le serpentin longitudinal.

19. Dispositif selon la revendication 18, dans lequel le fil métallique de structure allongée faisant office de partie centrale comprend une géométrie non circulaire en coupe transversale, configurée pour conférer des caractéristiques de flexion non homogènes à la portion distale flexible de l'assemblage d'ancrage.

20. Dispositif selon la revendication 19, dans lequel la géométrie non circulaire en coupe transversale comprend une configuration de forme rectangulaire en coupe transversale.

21. Dispositif selon la revendication 1, dans lequel une extrémité proximale de la portion distale flexible (2) de l'assemblage d'ancrage est couplée de manière amovible à une extrémité distale de la portion médiane (4) incurvée de manière préalable.

22. Dispositif selon la revendication 21, dans lequel l'extrémité proximale de la portion distale flexible (2) de l'assemblage d'ancrage est couplée de manière amovible à l'extrémité distale de la portion médiane (4) incurvée de manière préalable, en utilisant un accessoire de verrouillage mécanique.

23. Dispositif selon la revendication 1, dans lequel l'assemblage d'ancrage et l'aiguille (40) sont configurés pour créer la voie (32) apte à s'élargir qui possède une géométrie, par rapport à la paroi du vaisseau sanguin, telle que, lors du retrait de structures à partir de la voie (32) apte à s'élargir, la pression sanguine qui s'exerce sur la paroi du vaisseau fait en sorte que les portions chevauchantes de tissu s'affaissent les unes contre les autres et procurent une étanchéité automatique à la voie (32) apte à s'élargir.

24. Dispositif selon la revendication 1, dans lequel la structure de réception d'aiguille possédant une configuration en forme de selle, qui procure un encapsulage au moins partiel comprend une portion en forme d'élément tubulaire, découpée au laser.

25. Dispositif selon la revendication 1, dans lequel la structure de réception d'aiguille possédant une configuration en forme de selle, qui procure un encapsulage au moins partiel comprend une portion en forme d'élément tubulaire, obtenue par voie mécanique.

26. Dispositif selon la revendication 1, dans lequel la structure de réception d'aiguille possédant une configuration en forme de selle, qui procure un encapsulage au moins partiel possède une géométrie qui est configurée pour encapsuler environ la moitié de la géométrie superficielle d'une extrémité distale de l'aiguille, lorsque celle-ci est raccordée en interface à l'aiguille (40).
